(19) Europäisches Patentamt
European Patent Office
Office européen des brevets

(11) EP 1 230 928 A1

(12) EUROPEAN PATENT APPLICATION
published in accordance with Art. 158(3) EPC

(43) Date of publication:
**14.08.2002 Bulletin 2002/33**

(21) Application number: **00976321.0**

(22) Date of filing: **17.11.2000**

(51) Int Cl.[7]: **A61K 38/17**, A61P 5/40, A61P 3/04

(86) International application number:
**PCT/JP00/08119**

(87) International publication number:
**WO 01/35984 (25.05.2001 Gazette 2001/21)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **18.11.1999 JP 32790099**
**26.09.2000 JP 2000297073**

(71) Applicant: **Takeda Chemical Industries, Ltd.**
**Osaka-shi, Osaka 541-0045 (JP)**

(72) Inventors:
- **KITADA, Chieko**
**Sakai-shi, Osaka 590-0073 (JP)**
- **MATSUMOTO, Hirokazu**
**Tsukuba-shi, Ibaraki 305-0821 (JP)**
- **HINUMA, Shuji**
**Tsukuba-shi, Ibaraki 305-0821 (JP)**

(74) Representative: **Lewin, John Harvey**
**Takeda Patent Office,**
**11-12 Charles II Street**
**London SW1Y 4QU (GB)**

# (54) USE OF PEPTIDE

(57) The ligand polypeptides of the present invention have activity to regulate CRH secretion, and thus are useful as CRH secretion regulators for the relief, prevention or treatment of various diseases related to CRH secretion, such as hypoaldosteronism, hypocortisolemia, secondary and chronic adrenocorticism, Addison's disease (weakness, nausea, pigmentation, hypogonadism, alopecia, and hypotension), adrenal dysfunction, and obesity, and also as analgesics.

EP 1 230 928 A1

[Sequence Listing]

<110> Takeda Chemical Industries, Ltd.

<120> Use of Peptide

<130> 2671WOOP

<150> JP 11-327900

<151> 1999-11-18

<150> JP 12-297073

<151> 2000-09-26

<160> 49

<210> 1

<211> 98

<212> PRT

<213> Bovine

<400> 1

Met Lys Ala Val Gly Ala Trp Leu Leu Cys Leu Leu Leu Leu Gly Leu
1 5 10 15

Ala Leu Gln Gly Ala Ala Ser Arg Ala His Gln His Ser Met Glu Ile
20 25 30

Arg Thr Pro Asp Ile Asn Pro Ala Trp Tyr Ala Gly Arg Gly Ile Arg
35 40 45

Pro Val Gly Arg Phe Gly Arg Arg Arg Ala Ala Pro Gly Asp Gly Pro
50 55 60

Arg Pro Gly Pro Arg Arg Val Pro Ala Cys Phe Arg Leu Glu Gly Gly
65 70 75 80

Ala Glu Pro Ser Arg Ala Leu Pro Gly Arg Leu Thr Ala Gln Leu Val
85 90 95

Gln Glu

98

<210> 2

<211> 294

<212> DNA

<213> Bovine

<400> 2

ATGAAGGCGG TGGGGGCCTG GCTCCTCTGC CTGCTGCTGC TGGGCCTGGC CCTGCAGGGG 60

GCTGCCAGCA GAGCCCACCA GCACTCCATG GAGATCCGCA CCCCCGACAT CAACCCTGCC 120

TGGTACGCRG GCCGTGGGAT CCGGCCCGTG GGCCGCTTCG GCCGGCGAAG AGCTGCCCYG 180

GGGGACGGAC CCAGGCCTGG CCCCCGGCGT GTGCCGGCCT GCTTCCGCCT GGAAGGCGGY 240

GCTGAGCCCT CCCGAGCCCT CCCGGGGCGG CTGACGGCCC AGCTGGTCCA GGAA 294

<210> 3

<211> 31

<212> PRT

<213> Bovine

<400> 3

Ser Arg Ala His Gln His Ser Met Glu Ile Arg Thr Pro Asp Ile Asn

1 5 10 15

Pro Ala Trp Tyr Ala Gly Arg Gly Ile Arg Pro Val Gly Arg Phe

20 25 30 31

<210> 4

<211> 32

<212> PRT

<213> Bovine

<400> 4

Ser Arg Ala His Gln His Ser Met Glu Ile Arg Thr Pro Asp Ile Asn
1 5 10 15
Pro Ala Trp Tyr Ala Gly Arg Gly Ile Arg Pro Val Gly Arg Phe Gly
20 25 30 32

<210> 5
<211> 33
<212> PRT
<213> Bovine
<400> 5

Ser Arg Ala His Gln His Ser Met Glu Ile Arg Thr Pro Asp Ile Asn
1 5 10 15
Pro Ala Trp Tyr Ala Gly Arg Gly Ile Arg Pro Val Gly Arg Phe Gly
20 25 30
Arg
33

<210> 6
<211> 20
<212> PRT
<213> Bovine
<400> 6

Thr Pro Asp Ile Asn Pro Ala Trp Tyr Ala Gly Arg Gly Ile Arg Pro
1 5 10 15
Val Gly Arg Phe
20

<210> 7
<211> 21

<212> PRT

<213> Bovine

<400> 7

Thr Pro Asp Ile Asn Pro Ala Trp Tyr Ala Gly Arg Gly Ile Arg Pro
1 5 10 15

Val Gly Arg Phe Gly
20 21

<210> 8

<211> 22

<212> PRT

<213> Bovine

<400> 8

Thr Pro Asp Ile Asn Pro Ala Trp Tyr Ala Gly Arg Gly Ile Arg Pro
1 5 10 15

Val Gly Arg Phe Gly Arg
20 22

<210> 9

<211> 93

<212> DNA

<213> Bovine

<400> 9

AGCAGAGCCC ACCAGCACTC CATGGAGATC CGCACCCCCG ACATCAACCC TGCCTGGTAC 60

GCRGGCCGTG GGATCCGGCC CGTGGGCCGC TTC 93

<210> 10

<211> 96

<212> DNA

<213> Bovine

<400> 10

AGCAGAGCCC ACCAGCACTC CATGGAGATC CGCACCCCCG ACATCAACCC TGCCTGGTAC 60

GCRGGCCGTG GGATCCGGCC CGTGGGCCGC TTCGGC 96

<210> 11

<211> 99

<212> DNA

<213> Bovine

<400> 11

AGCAGAGCCC ACCAGCACTC CATGGAGATC CGCACCCCCG ACATCAACCC TGCCTGGTAC 60

GCRGGCCGTG GGATCCGGCC CGTGGGCCGC TTCGGCCGG 99

<210> 12

<211> 60

<212> DNA

<213> Bovine

<400> 12

ACCCCCGACA TCAACCCTGC CTGGTACGCR GGCCGTGGGA TCCGGCCCGT GGGCCGCTTC 60

<210> 13

<211> 63

<212> DNA

<213> Bovine

<400> 13

ACCCCCGACA TCAACCCTGC CTGGTACGCR GGCCGTGGGA TCCGGCCCGT GGGCCGCTTC 60

GGC 63

<210> 14

<211> 66

<212> DNA

<213> Bovine

<400> 14

ACCCCCGACA TCAACCCTGC CTGGTACGCR GGCCGTGGGA TCCGGCCCGT GGGCCGCTTC 60

GGCCGG 66

<210> 15

<211> 98

<212> PRT

<213> Bovine

<400> 15

Met Lys Ala Val Gly Ala Trp Leu Leu Cys Leu Leu Leu Leu Gly Leu
1 5 10 15

Ala Leu Gln Gly Ala Ala Ser Arg Ala His Gln His Ser Met Glu Ile
20 25 30

Arg Thr Pro Asp Ile Asn Pro Ala Trp Tyr Ala Gly Arg Gly Ile Arg
35 40 45

Pro Val Gly Arg Phe Gly Arg Arg Arg Ala Ala Leu Gly Asp Gly Pro
50 55 60

Arg Pro Gly Pro Arg Arg Val Pro Ala Cys Phe Arg Leu Glu Gly Gly
65 70 75 80

Ala Glu Pro Ser Arg Ala Leu Pro Gly Arg Leu Thr Ala Gln Leu Val
85 90 95

Gln Glu
98

<210> 16

<211> 83

<212> PRT

<213> Rat

<400> 16

Met Ala Leu Lys Thr Trp Leu Leu Cys Leu Leu Leu Leu Ser Leu Val
1 5 10 15

Leu Pro Gly Ala Ser Ser Arg Ala His Gln His Ser Met Glu Thr Arg
20 25 30

Thr Pro Asp Ile Asn Pro Ala Trp Tyr Thr Gly Arg Gly Ile Arg Pro
35 40 45

Val Gly Arg Phe Gly Arg Arg Arg Ala Thr Pro Arg Asp Val Thr Gly
50 55 60

Leu Gly Gln Leu Ser Cys Leu Pro Leu Asp Gly Arg Thr Lys Phe Ser
65 70 75 80

Gln Arg Gly
83

<210> 17

<211> 249

<212> DNA

<213> Rat

<400> 17

ATGGCCCTGA AGACGTGGCT TCTGTGCTTG CTGCTGCTAA GCTTGGTCCT CCCAGGGGCT 60
TCCAGCCGAG CCCACCAGCA CTCCATGGAG ACAAGAACCC CTGATATCAA TCCTGCCTGG 120
TACACGGGCC GCGGGATCAG GCCTGTGGGC CGCTTCGGCA GGAGAAGGGC AACCCCGAGG 180
GATGTCACTG GACTTGGCCA ACTCAGCTGC CTCCCACTGG ATGGACGCAC CAAGTTCTCT 240
CAGCGTGGA 249

<210> 18

<211> 31

<212> PRT

<213> Rat

<400> 18

Ser Arg Ala His Gln His Ser Met Glu Thr Arg Thr Pro Asp Ile Asn
1 5 10 15

Pro Ala Trp Tyr Thr Gly Arg Gly Ile Arg Pro Val Gly Arg Phe
20 25 30 31

<210> 19

<211> 32

<212> PRT

<213> Rat

<400> 19

Ser Arg Ala His Gln His Ser Met Glu Thr Arg Thr Pro Asp Ile Asn
1 5 10 15

Pro Ala Trp Tyr Thr Gly Arg Gly Ile Arg Pro Val Gly Arg Phe Gly
20 25 30 32

<210> 20

<211> 33

<212> PRT

<213> Rat

<400> 20

Ser Arg Ala His Gln His Ser Met Glu Thr Arg Thr Pro Asp Ile Asn
1 5 10 15

Pro Ala Trp Tyr Thr Gly Arg Gly Ile Arg Pro Val Gly Arg Phe Gly
20 25 30

Arg

33

<210> 21

<211> 20

<212> PRT

<213> Rat

<400> 21

Thr Pro Asp Ile Asn Pro Ala Trp Tyr Thr Gly Arg Gly Ile Arg Pro

1 5 10 15

Val Gly Arg Phe

20

<210> 22

<211> 21

<212> PRT

<213> Rat

<400> 22

Thr Pro Asp Ile Asn Pro Ala Trp Tyr Thr Gly Arg Gly Ile Arg Pro

1 5 10 15

Val Gly Arg Phe Gly

20 21

<210> 23

<211> 22

<212> PRT

<213> Rat

<400> 23

Thr Pro Asp Ile Asn Pro Ala Trp Tyr Thr Gly Arg Gly Ile Arg Pro

1 5 10 15

Val Gly Arg Phe Gly Arg

20 22

<210> 24

<211> 93

<212> DNA

<213> Rat

<400> 24

AGCCGAGCCC ACCAGCACTC CATGGAGACA AGAACCCCTG ATATCAATCC TGCCTGGTAC 60

ACGGGCCGCG GGATCAGGCC TGTGGGCCGC TTC 93

<210> 25

<211> 96

<212> DNA

<213> Rat

<400> 25

AGCCGAGCCC ACCAGCACTC CATGGAGACA AGAACCCCTG ATATCAATCC TGCCTGGTAC 60

ACGGGCCGCG GGATCAGGCC TGTGGGCCGC TTCGGC 96

<210> 26

<211> 99

<212> DNA

<213> Rat

<400> 26

AGCCGAGCCC ACCAGCACTC CATGGAGACA AGAACCCCTG ATATCAATCC TGCCTGGTAC 60

ACGGGCCGCG GGATCAGGCC TGTGGGCCGC TTCGGCAGG 99

<210> 27

<211> 60

<212> DNA

<213> Rat

<400> 27

ACCCCTGATA TCAATCCTGC CTGGTACACG GGCCGCGGGA TCAGGCCTGT GGGCCGCTTC 60

<210> 28

<211> 63

<212> DNA

<213> Rat

<400> 28

ACCCCTGATA TCAATCCTGC CTGGTACACG GGCCGCGGGA TCAGGCCTGT GGGCCGCTTC 60

GGC 63

<210> 29

<211> 66

<212> DNA

<213> Rat

<400> 29

ACCCCTGATA TCAATCCTGC CTGGTACACG GGCCGCGGGA TCAGGCCTGT GGGCCGCTTC 60

GGCAGG 66

<210> 30

<211> 87

<212> PRT

<213> Human

<400> 30

Met Lys Val Leu Arg Ala Trp Leu Leu Cys Leu Leu Met Leu Gly Leu

1 5 10 15

Ala Leu Arg Gly Ala Ala Ser Arg Thr His Arg His Ser Met Glu Ile

20 25 30

Arg Thr Pro Asp Ile Asn Pro Ala Trp Tyr Ala Ser Arg Gly Ile Arg

35 40 45

Pro Val Gly Arg Phe Gly Arg Arg Arg Ala Thr Leu Gly Asp Val Pro

50 55 60

Lys Pro Gly Leu Arg Pro Arg Leu Thr Cys Phe Pro Leu Glu Gly Gly

65 70 75 80

Ala Met Ser Ser Gln Asp Gly

85 87

<210> 31

<211> 261

<212> DNA

<213> Human

<400> 31

ATGAAGGTGC TGAGGGCCTG GCTCCTGTGC CTGCTGATGC TGGGCCTGGC CCTGCGGGGA 60

GCTGCAAGTC GTACCCATCG GCACTCCATG GAGATCCGCA CCCCTGACAT CAATCCTGCC 120

TGGTACGCCA GTCGCGGGAT CAGGCCTGTG GGCCGCTTCG GTCGGAGGAG GGCAACCCTG 180

GGGGACGTCC CCAAGCCTGG CCTGCGACCC CGGCTGACCT GCTTCCCCCT GGAAGGCGGT 240

GCTATGTCGT CCCAGGATGG C 261

<210> 32

<211> 31

<212> PRT

<213> Human

<400> 32

Ser Arg Thr His Arg His Ser Met Glu Ile Arg Thr Pro Asp Ile Asn

1 5 10 15

Pro Ala Trp Tyr Ala Ser Arg Gly Ile Arg Pro Val Gly Arg Phe

20 25 30 31

<210> 33

<211> 32

<212> PRT

<213> Human

<400> 33

Ser Arg Thr His Arg His Ser Met Glu Ile Arg Thr Pro Asp Ile Asn

1 5 10 15

Pro Ala Trp Tyr Ala Ser Arg Gly Ile Arg Pro Val Gly Arg Phe Gly

20 25 30 32

<210> 34

<211> 33

<212> PRT

<213> Human

<400> 34

Ser Arg Thr His Arg His Ser Met Glu Ile Arg Thr Pro Asp Ile Asn

1 5 10 15

Pro Ala Trp Tyr Ala Ser Arg Gly Ile Arg Pro Val Gly Arg Phe Gly

20 25 30

Arg

33

<210> 35

<211> 20

<212> PRT

<213> Human

<400> 35

Thr Pro Asp Ile Asn Pro Ala Trp Tyr Ala Ser Arg Gly Ile Arg Pro
1 5 10 15

Val Gly Arg Phe
20

<210> 36

<211> 21

<212> PRT

<213> Human

<400> 36

Thr Pro Asp Ile Asn Pro Ala Trp Tyr Ala Ser Arg Gly Ile Arg Pro
1 5 10 15

Val Gly Arg Phe Gly
20 21

<210> 37

<211> 22

<212> PRT

<213> Human

<400> 37

Thr Pro Asp Ile Asn Pro Ala Trp Tyr Ala Ser Arg Gly Ile Arg Pro
1 5 10 15

Val Gly Arg Phe Gly Arg
20 22

<210> 38

<211> 93

<212> DNA

<213> Human

<400> 38

AGTCGTACCC ATCGGCACTC CATGGAGATC CGCACCCCTG ACATCAATCC TGCCTGGTAC 60

GCCAGTCGCG GGATCAGGCC TGTGGGCCGC TTC 93

<210> 39

<211> 96

<212> DNA

<213> Human

<400> 39

AGTCGTACCC ATCGGCACTC CATGGAGATC CGCACCCCTG ACATCAATCC TGCCTGGTAC 60

GCCAGTCGCG GGATCAGGCC TGTGGGCCGC TTCGGT 96

<210> 40

<211> 99

<212> DNA

<213> Human

<400> 40

AGTCGTACCC ATCGGCACTC CATGGAGATC CGCACCCCTG ACATCAATCC TGCCTGGTAC 60

GCCAGTCGCG GGATCAGGCC TGTGGGCCGC TTCGGTCGG 99

<210> 41

<211> 60

<212> DNA

<213> Human

<400> 41

ACCCCTGACA TCAATCCTGC CTGGTACGCC AGTCGCGGGA TCAGGCCTGT GGGCCGCTTC 60

<210> 42

<211> 63

<212> DNA

<213> Human

<400> 42

ACCCCTGACA TCAATCCTGC CTGGTACGCC AGTCGCGGGA TCAGGCCTGT GGGCCGCTTC 60

GGT 63

<210> 43

<211> 66

<212> DNA

<213> Human

<400> 43

ACCCCTGACA TCAATCCTGC CTGGTACGCC AGTCGCGGGA TCAGGCCTGT GGGCCGCTTC 60

GGTCGG 66

<210> 44

<211> 31

<212> PRT

<213> Unknown

<220>

<221>

<223> Xaa on the 3rd position means Thr or Ala, Xaa on the 5th position means Arg or Gln, Xaa on the 10th position means Ile or Thr, Xaa on the 21st position means Thr or Ala, Xaa on the 22nd position means Gly or Ser.

<400> 44

Ser Arg Xaa His Xaa His Ser Met Glu Xaa Arg Thr Pro Asp Ile Asn
1 5 10 15

Pro Ala Trp Tyr Xaa Xaa Arg Gly Ile Arg Pro Val Gly Arg Phe
20 25 30 31

<210> 45

<211> 20

<212> PRT

<213> Unknown

<220>

<221>

<223> Xaa on the 10th position means Thr or Ala, Xaa on the 11th position means Gly or Ser.

<400> 45

Thr Pro Asp Ile Asn Pro Ala Trp Tyr Xaa Xaa Arg Gly Ile Arg Pro
1 5 10 15

Val Gly Arg Phe
20

<210> 46

<211> 370

<212> PRT

<213> Human

<400> 46

Met Ala Ser Ser Thr Thr Arg Gly Pro Arg Val Ser Asp Leu Phe Ser
1 5 10 15

Gly Leu Pro Pro Ala Val Thr Thr Pro Ala Asn Gln Ser Ala Glu Ala
20 25 30

Ser Ala Gly Asn Gly Ser Val Ala Gly Ala Asp Ala Pro Ala Val Thr
35 40 45

Pro Phe Gln Ser Leu Gln Leu Val His Gln Leu Lys Gly Leu Ile Val
50 55 60

Leu Leu Tyr Ser Val Val Val Val Val Gly Leu Val Gly Asn Cys Leu
65 70 75 80
Leu Val Leu Val Ile Ala Arg Val Arg Arg Leu His Asn Val Thr Asn
85 90 95
Phe Leu Ile Gly Asn Leu Ala Leu Ser Asp Val Leu Met Cys Thr Ala
100 105 110
Cys Val Pro Leu Thr Leu Ala Tyr Ala Phe Glu Pro Arg Gly Trp Val
115 120 125
Phe Gly Gly Gly Leu Cys His Leu Val Phe Phe Leu Gln Pro Val Thr
130 135 140
Val Tyr Val Ser Val Phe Thr Leu Thr Thr Ile Ala Val Asp Arg Tyr
145 150 155 160
Val Val Leu Val His Pro Leu Arg Arg Arg Ile Ser Leu Arg Leu Ser
165 170 175
Ala Tyr Ala Val Leu Ala Ile Trp Ala Leu Ser Ala Val Leu Ala Leu
180 185 190
Pro Ala Ala Val His Thr Tyr His Val Glu Leu Lys Pro His Asp Val
195 200 205
Arg Leu Cys Glu Glu Phe Trp Gly Ser Gln Glu Arg Gln Arg Gln Leu
210 215 220
Tyr Ala Trp Gly Leu Leu Leu Val Thr Tyr Leu Leu Pro Leu Leu Val
225 230 235 240
Ile Leu Leu Ser Tyr Val Arg Val Ser Val Lys Leu Arg Asn Arg Val
245 250 255
Val Pro Gly Cys Val Thr Gln Ser Gln Ala Asp Trp Asp Arg Ala Arg
260 265 270

Arg Arg Arg Thr Phe Cys Leu Leu Val Val Val Val Val Val Phe Ala
275 280 285

Val Cys Trp Leu Pro Leu His Val Phe Asn Leu Leu Arg Asp Leu Asp
290 295 300

Pro His Ala Ile Asp Pro Tyr Ala Phe Gly Leu Val Gln Leu Leu Cys
305 310 315 320

His Trp Leu Ala Met Ser Ser Ala Cys Tyr Asn Pro Phe Ile Tyr Ala
325 330 335

Trp Leu His Asp Ser Phe Arg Glu Glu Leu Arg Lys Leu Leu Val Ala
340 345 350

Trp Pro Arg Lys Ile Ala Pro His Gly Gln Asn Met Thr Val Ser Val
355 360 365

Val Ile
370

<210> 47

<211> 1110

<212> DNA

<213> Human

<400> 47

ATGGCCTCAT CGACCACTCG GGGCCCCAGG GTTTCTGACT TATTTTCTGG GCTGCCGCCG 60

GCGGTCACAA CTCCCGCCAA CCAGAGCGCA GAGGCCTCGG CGGGCAACGG GTCGGTGGCT 120

GGCGCGGACG CTCCAGCCGT CACGCCCTTC CAGAGCCTGC AGCTGGTGCA TCAGCTGAAG 180

GGGCTGATCG TGCTGCTCTA CAGCGTCGTG GTGGTCGTGG GGCTGGTGGG CAACTGCCTG 240

CTGGTGCTGG TGATCGCGCG GGTGCGCCGG CTGCACAACG TGACGAACTT CCTCATCGGC 300

AACCTGGCCT TGTCCGACGT GCTCATGTGC ACCGCCTGCG TGCCGCTCAC GCTGGCCTAT 360

GCCTTCGAGC CACGCGGCTG GGTGTTCGGC GGCGGCCTGT GCCACCTGGT CTTCTTCCTG 420

CAGCCGGTCA CCGTCTATGT GTCGGTGTTC ACGCTCACCA CCATCGCAGT GGACCGCTAC 480
GTCGTGCTGG TGCACCCGCT GAGGCGGCGC ATCTCGCTGC GCCTCAGCGC CTACGCTGTG 540
CTGGCCATCT GGGCGCTGTC CGCGGTGCTG GCGCTGCCCG CCGCCGTGCA CACCTATCAC 600
GTGGAGCTCA AGCCGCACGA CGTGCGCCTC TGCGAGGAGT TCTGGGGCTC CCAGGAGCGC 660
CAGCGCCAGC TCTACGCCTG GGGGCTGCTG CTGGTCACCT ACCTGCTCCC TCTGCTGGTC 720
ATCCTCCTGT CTTACGTCCG GGTGTCAGTG AAGCTCCGCA ACCGCGTGGT GCCGGGCTGC 780
GTGACCCAGA GCCAGGCCGA CTGGGACCGC GCTCGGCGCC GGCGCACCTT CTGCTTGCTG 840
GTGGTGGTCG TGGTGGTGTT CGCCGTCTGC TGGCTGCCGC TGCACGTCTT CAACCTGCTG 900
CGGGACCTCG ACCCCCACGC CATCGACCCT TACGCCTTTG GGCTGGTGCA GCTGCTCTGC 960
CACTGGCTCG CCATGAGTTC GGCCTGCTAC AACCCCTTCA TCTACGCCTG GCTGCACGAC 1020
AGCTTCCGCG AGGAGCTGCG CAAACTGTTG GTCGCTTGGC CCCGCAAGAT AGCCCCCCAT 1080
GGCCAGAATA TGACCGTCAG CGTGGTCATC 1110

<210> 48

<211> 370

<212> PRT

<213> Rat

<400> 48

Met Thr Ser Leu Pro Pro Gly Thr Thr Gly Asp Pro Asp Leu Phe Ser
1 5 10 15
Gly Pro Ser Pro Ala Gly Ser Thr Pro Ala Asn Gln Ser Ala Glu Ala
20 25 30
Ser Glu Ser Asn Val Ser Ala Thr Val Pro Arg Ala Ala Ala Val Thr
35 40 45
Pro Phe Gln Ser Leu Gln Leu Val His Gln Leu Lys Gly Leu Ile Val
50 55 60
Met Leu Tyr Ser Ile Val Val Val Val Gly Leu Val Gly Asn Cys Leu

65 70 75 80
Leu Val Leu Val Ile Ala Arg Val Arg Arg Leu His Asn Val Thr Asn
85 90 95
Phe Leu Ile Gly Asn Leu Ala Leu Ser Asp Val Leu Met Cys Ala Ala
100 105 110
Cys Val Pro Leu Thr Leu Ala Tyr Ala Phe Glu Pro Arg Gly Trp Val
115 120 125
Phe Gly Gly Gly Leu Cys His Leu Val Phe Phe Leu Gln Pro Val Thr
130 135 140
Val Tyr Val Ser Val Phe Thr Leu Thr Thr Ile Ala Val Asp Arg Tyr
145 150 155 160
Val Val Leu Val His Pro Leu Arg Arg Arg Ile Ser Leu Lys Leu Ser
165 170 175
Ala Tyr Ala Val Leu Gly Ile Trp Ala Leu Ser Ala Val Leu Ala Leu
180 185 190
Pro Ala Ala Val His Thr Tyr His Val Glu Leu Lys Pro His Asp Val
195 200 205
Arg Leu Cys Glu Glu Phe Trp Gly Ser Gln Glu Arg Gln Arg Gln Ile
210 215 220
Tyr Ala Trp Gly Leu Leu Leu Gly Thr Tyr Leu Leu Pro Leu Leu Ala
225 230 235 240
Ile Leu Leu Ser Tyr Val Arg Val Ser Val Lys Leu Arg Asn Arg Val
245 250 255
Val Pro Gly Ser Val Thr Gln Ser Gln Ala Asp Trp Asp Arg Ala Arg
260 265 270
Arg Arg Arg Thr Phe Cys Leu Leu Val Val Val Val Val Val Phe Ala

275 280 285

Leu Cys Trp Leu Pro Leu His Ile Phe Asn Leu Leu Arg Asp Leu Asp

290 295 300

Pro Arg Ala Ile Asp Pro Tyr Ala Phe Gly Leu Val Gln Leu Leu Cys

305 310 315 320

His Trp Leu Ala Met Ser Ser Ala Cys Tyr Asn Pro Phe Ile Tyr Ala

325 330 335

Trp Leu His Asp Ser Phe Arg Glu Glu Leu Arg Lys Met Leu Leu Ser

340 345 350

Trp Pro Arg Lys Ile Val Pro His Gly Gln Asn Met Thr Val Ser Val

355 360 365

Val Ile

370

<210> 49

<211> 1110

<212> DNA

<213> Rat

<400> 49

ATGACCTCAC TGCCCCCTGG AACCACTGGG GACCCCGATT TGTTTTCTGG GCCGTCGCCA 60

GCCGGCTCCA CTCCAGCCAA CCAGAGTGCA GAGGCTTCAG AGAGCAATGT GTCTGCGACG 120

GTTCCCAGAG CTGCAGCAGT CACGCCGTTC CAGAGCCTGC AACTAGTGCA CCAGCTGAAG 180

GGACTGATCG TGATGCTGTA CAGCATCGTG GTGGTCGTGG GTCTGGTGGG CAACTGCCTT 240

CTTGTGCTGG TGATCGCGCG CGTGCGCCGG CTGCACAACG TGACCAACTT CCTCATCGGC 300

AACCTGGCCT TGTCCGATGT GCTCATGTGT GCCGCCTGTG TGCCTCTCAC GCTGGCCTAC 360

GCCTTTGAAC CTCGTGGCTG GGTGTTCGGT GGAGGCCTGT GCCACCTTGT TTTCTTCCTG 420

CAGCCGGTCA CCGTCTACGT ATCGGTGTTC ACACTCACCA CAATCGCTGT GGACCGCTAT 480

```
GTGGTTCTGG TGCACCCGCT ACGTCGGCGC ATTTCACTGA AGCTCAGCGC CTACGCTGTG  540
CTGGGCATCT GGGCTCTATC TGCAGTGCTG GCGCTGCCGG CCGCGGTGCA CACCTACCAT  600
GTAGAGCTCA AGCCCCACGA CGTGCGCCTC TGCGAGGAGT TCTGGGGTTC GCAGGAGCGC  660
CAGAGACAGA TCTATGCCTG GGGGCTGCTG CTGGGCACCT ATTTGCTCCC CCTGCTGGCC  720
ATTCTCCTGT CTTACGTCCG GGTGTCGGTG AAGTTGCGGA ACCGCGTGGT GCCTGGCAGC  780
GTGACCCAGA GCCAGGCTGA CTGGGACCGA GCGCGTCGCC GTCGCACTTT CTGCCTGCTG  840
GTGGTGGTGG TGGTCGTGTT CGCGCTCTGC TGGCTGCCTC TGCACATTTT CAACCTTCTG  900
CGGGACCTGG ACCCGCGTGC CATCGACCCC TACGCCTTCG GGCTGGTGCA GCTCCTCTGC  960
CACTGGCTTG CCATGAGCTC CGCCTGCTAC AACCCCTTCA TCTATGCGTG GCTGCACGAC 1020
AGCTTCCGAG AGGAGCTACG CAAGATGCTT CTGTCTTGGC CCCGCAAGAT CGTGCCTCAT 1080
GGCCAGAATA TGACCGTCAG TGTGGTCATC                                  1110
```

## Description

### TECHNICAL FIELD

The present invention relates to use of physiologically active peptides, and in particular to Corticotropin-Releasing Hormone (CRH) secretion regulators or the like comprising ligand polypeptides for G protein-coupled receptor (hereinafter sometimes referred to as "receptor") proteins.

### BACKGROUND ART

Most hormones or neurotransmitters regulate the functions of an organism through specific receptors on cell membranes. Most of these receptors transduce intracellular signals through the activation of coupled guanine nucleotide-binding proteins (hereinafter sometimes referred to as "G proteins"). These receptors have a shared structure with seven transmembrane domains, and are thus referred to as G protein-coupled receptors or seven-pass transmembrane receptors (7TMR).

Known examples of such G protein-coupled receptor proteins include human receptor proteins encoded by the phGR3 (also referred to as GPR10) gene (*Genomics,* Vol. 29, p. 335 (1995)) and the corresponding rat receptor protein UHR-1 (*Biochem. Biophy. Res. Commun.,* Vol. 209, p. 606 (1995)).

PrRP (*Nature,* Vol. 393, pp. 272-276 (1998)) is a known physiologically active peptide that functions as a ligand for the aforementioned phGR3 and UHR-1.

PrRP has specific prolactin-releasing activity in the anterior pituitary *(Nature,* Vol. 393, pp. 272-276 (1998), and *Biochem. Biophy. Res. Commun.*, Vol. 259, pp. 321-324 (1999)), but the activity of PrRP in the brain is unknown. The endogenous regulatory hormones which regulate corticotropin-releasing hormone (CRH), a hypothalamic hormone, are also unknown at present.

### DISCLOSURE OF THE INVENTION

As a result of extensive research to address the aforementioned issues, the inventors first prepared two types of monoclonal antibodies specific to PrRP with different recognition sites, and prepared an assay system (Sandwich-EIA) highly sensitive to PrRP (WO99-60112). The use of this assay system to explore the tissue distribution of PrRP in rats revealed its widespread distribution in the hypothalamus and the like, as already reported *(Biochemical and Biophysical Research Communications,* Vol. 257, p. 264 (1999)), and confirmed the wide distribution of PrRP in the brain. As a result of further extensive research, the inventors completed the present invention upon the discovery that PrRP was unexpectedly detected in cerebrospinal fluid in concentrations 5 to 6 times greater than in the blood, with activity to regulate release of CRH.

Specifically, the present invention relates to:

(1) a corticotropin-releasing hormone (CRH) secretion regulator, comprising a ligand peptide for a G protein-coupled receptor protein, or an amide thereof, ester thereof, or salt thereof;
(2) a corticotropin-releasing hormone secretion regulator according to (1) above, wherein the ligand peptide for a G protein-coupled receptor protein, or amide thereof, ester thereof, or salt thereof comprises a polypeptide or amide thereof, ester thereof, or salt thereof comprising an amino acid sequence that is the same as or substantially the same as the amino acid sequence represented by SEQ ID NO. 44 or 45;
(3) a corticotropin-releasing hormone secretion regulator according to (2) above, wherein the amino acid sequence in SEQ ID NO. 44 is the amino acid sequence represented by SEQ ID NO. 3, 18 or 32;
(4) a corticotropin-releasing hormone secretion regulator according to (2) above, wherein the amino acid sequence represented by SEQ ID NO. 45 is the amino acid sequence represented by SEQ ID NO. 6, 21 or 35;
(5) a corticotropin-releasing hormone secretion regulator according to (1) above, wherein the G protein-coupled receptor protein comprises a protein comprising an amino acid sequence that is the same as or substantially the same as the amino acid sequence represented by SEQ ID NO. 46;
(6) a corticotropin-releasing hormone secretion regulator according to (1) above, which is a corticotropin-releasing hormone secretion promoter;
(7) a corticotropin-releasing hormone secretion regulator according to (1) above, which is a pharmaceutical agent for the treatment or prevention of hypoaldosteronism, hypocortisolemia, secondary or chronic hypoadrenocorticism, Addison's disease, adrenal dysfunction, or obesity;
(8) a corticotropin-releasing hormone secretion regulator, comprising a compound, or salt thereof, which alters binding between a G protein-coupled receptor protein and the ligand peptide for the G protein-coupled receptor protein;

(9) the use of a ligand peptide for a G protein-coupled receptor protein, or an amide thereof, ester thereof, or salt thereof for the production of drugs having activity to regulate the secretion of corticotropin-releasing hormones; and
(10) a method for regulating the secretion of corticotropin-releasing hormones, characterized by administrering a ligand peptide for a G protein-coupled receptor protein, or an amide thereof, ester thereof, or salt thereof to mammals.

BRIEF DESCRIPTION OF THE DRAWINGS

Figure 1 shows the changes in the concentration of ACTH in blood after the intraventricular administration of PrRP-31 (amide form of the peptide consisting of the amino acid sequence in SEQ ID NO. 32; same below) to ventricles of brain of rats. In the figure -○- represents the control (phosphate buffered physiological saline), -•- represents PrRP-31 (1 nmol), -Δ- represents PrRP-31 (3 nmol), and -■- represents PrRP-31 (10 nmol). The ACTH concentration is given as the measured value ± standard deviation. ** indicates there was a significant difference at the 1% level of significance (n=6-8), and * indicates there was a significant difference at the 5% level of significance (n=6-8);
Figure 2 shows the changes in the concentration of β-endorphin in blood after the intraventricular administration of PrRP-31 to ventricles of brain of rats. In the figure -○- represents the control (phosphate buffered physiological saline), -•- represents PrRP-31 (1 nmol), -Δ- represents PrRP-31 (3 nmol), and -■- represents PrRP-31 (10 nmol). The β-endorphin concentration is given as the measured value ± standard deviation. * indicates there was a significant difference at the 5% level of significance (n=6-8);
Figure 3 shows the changes in the concentration of ACTH in blood after the administration of anti-CRF IgG and normal rabbit IgG via the jugular vein, followed 30 minutes later by intraventricular administration of PrRP-31 to ventricles of brain of rats. In the figure-○- represents the administration of PrRP-31 (10 nmol) after administration of the control (phosphate buffered physiological saline), -Δ- represents the administration of PrRP-31 (10 nmol) after administration of normal rabbit IgG, and -•- represents the administration of PrRP-31 (10 nmol) after administration of anti-CRF IgG;
Figure 4 shows the changes in the concentration of β-endorphin in blood after the administration of anti-CRF IgG and normal rabbit IgG via the jugular vein, followed 30 minutes later by intraventricular administration of PrRP-31 to cerebral ventricles of rats. In the figure -○- represents the administration of PrRP-31 (10 nmol) after administration of the control (phosphate buffered physiological saline), -Δ- represents the administration of PrRP-31 (10 nmol) after administration of normal rabbit IgG, and -•- represents the administration of PrRP-31 (10 nmol) after administration of anti-CRF IgG. The β-endorphin concentration is given as the measured value ± standard deviation. * indicates there was a significant difference at the 5% level of significance (n=6-8);
Figure 5 shows the changes in the concentration of PrRP-31 in the cerebrospinal fluid as a result of water-immersion stress loading;
Figure 6 shows the changes in the concentration of ACTH in blood as a result of water-immersion stress. The ACTH concentration is given as the measured value ± standard deviation. ** indicates there was a significant difference at the 1% level of significance (n=7-8); and
Figure 7 shows the changes in the concentration of ACTH in blood after the administration of α-helical CRF and physiological saline via the jugular vein, followed 15 minutes later by intraventricular administration of PrRP-31 to cerebral ventricles of rats. In the figure, -•- represents the administration of PrRP-31 (10 nmol) after administration of the physiological saline, and -○- represents the administration of PrRP-31 (10 nmol) after administration of α-helical CRF. The ACTH concentration is given as the measured value ± standard deviation. ** indicates there was a significant difference at the 1% level of significance (n=7-8).* indicates there was a significant difference at the 5% level of significance (n=7-8).

BEST MODE FOR CARRYING OUT THE INVENTION

The abbreviations for bases, amino acids, and the like in the Specification and drawings are based on the abbreviations authorized by the IUPAC-IUB Commission on Biochemical Nomenclature, or other abbreviations commonly used in the field, examples of which are given below. Unless otherwise indicated, amino acid optical isomers are the L form.

DNA: deoxyribonucleic acid
cDNA: complementary deoxyribonucleic acid
A: adenine

T: thymine
G: guanine
C: cytosine
RNA: ribonucleic acid
mRNA: messenger ribonucleic acid
ATP: adenosine triphosphate
EDTA: ethylenediaminetetracetic acid
SDS: sodium dodecylsulfate
EIA: enzyme immunoassay
Gly or G: glycine
Ala or A: alanine
Val or V: valine
Leu or L: leucine
Ile or I: isoleucine
Ser or S: serine
Thr or T: threonine
Cys or C: cystine
Met or M: methionine
Glu or E: glutamic acid
Asp or D: aspartic acid
Lys or K: lysine
Arg or R: arginine
His or H: histidine
Phe or F: phenylalanine
Tyr or Y: tyrosine
Trp or W: tryptophan
Pro or P: proline
Asn or N: asparagine
Gln or Q: glutamine
pGlu: pyroglutamic acid
Me: methyl group
Et: ethyl group
Bu: butyl group
Ph: phenyl group

Substituents, protective groups, and reagents used in the Specification are represented by the following symbols.

BHA: benzhydrylamine
pMBHA: p-methylbenzhydrylamine
Tos: p-toluenesulfonyl
CHO: formyl
HONB: N-hydroxy-5-norbornene-2,3-dicarboxyimide
OcHex: cyclohexyl ester
Bzl: benzyl
$Cl_2$-Bzl: dichlorobenzyl
Bom: benzyloxymethyl
Z: benzyloxycarbonyl
Br-Z: 2-bromobenzyloxycarbonyl
Boc: t-butyloxycarbonyl
DCM: dichloromethane
HOBt: 1-hydroxybenztriazole
DCC: N,N'-dicyclohexylcarbodiimide
TFA: trifluoroacetic acid
DIEA: diisopropylethylamine
Fmoc: N-9-fluorenylmethoxycarbonyl
DNP: dinitrophenyl
Bum: tert-butoxymethyl

Trt: trityl

As used in the specification, "substantially the same as" means that the polypeptide activity (such as the ligand-receptor binding activity), the CRH secretion-regulating activity of polypeptides (such as CRH secretion-promoting activity and CRH secretion-inhibiting activity), or the like is substantially the same. As such, an "amino acid sequence that is substantially the same" means amino acid sequences which may have mutations, provided that the polypeptide activity (such as the ligand-receptor binding activity) or the CRH secretion-regulating activity of polypeptides (such as CRH secretion-promoting activity and CRH secretion-inhibiting activity), or the like is substantially the same (is not significantly changed).

It is generally known that mutations such as substitutions, deletions, or insertions (additions) of amino acids in the amino acid sequence coding for polypeptides often do not bring about much (significant) change in the physiological or chemical properties of polypeptides. Examples of said substitutions include the substitution of a given amino acid with another amino acid having a similar character (characteristics). In general, substitution with amino acids of much more similar characteristics should result in less of change in the characteristics of the original unsubstituted polypeptide, which may be affected by such substitution.

Amino acids are classified on the basis of similarities in their characteristics as follows. The (i) nonpolar (hydrophobic) amino acids include alanine, leucine, isoleucine, valine, proline, phenylalanine, tryptophan, and methionines. The (ii) polar (neutral) amino acids include glycine, serine, threonine, cysteine, tyrosine, asparagine, and glutamine. The (iii) positively charged (basic) amino acids include arginine, lysine, and histidine. The (iv) negatively charged (acidic) amino acids include aspartic acid and glutamic acid.

"Substantially the same" substitutions of an amino acid in the target amino acid sequences in the specification may often be selected from other amino acids with similar characteristics among the class to which that amino acid belongs.

In the present invention, polypeptides (mutant polypeptides) obtained as a result of mutations in the amino acid sequence through substitutions, deletions, insertions or the like which do not bring about major (significant) changes in the physiological or chemical characteristics of the original (unmutated) polypeptide are regarded as being substantially the same as the original (unmutated) polypeptide having no such mutations. The amino acid sequences of such mutant polypeptides are also regarded as being substantially the same as the amino acid sequence of the original (unmutated) polypeptide.

The constituent amino acids of the polypeptides in the present invention may be either the D or L form, but the L form is usually preferred, unless otherwise specified.

The polypeptides in the present invention are ligand peptides for G protein-coupled receptor proteins, or amides thereof, esters thereof, or salts thereof, and are specifically ligand polypeptides for G protein-coupled receptor proteins, or amides thereof, esters thereof, or salts thereof. More specific examples include polypeptides or amides, esters, or salts thereof (hereinafter sometimes referred to simply as ligand polypeptides of the present invention or polypeptides of the present invention) having an amino acid sequence that is the same as or substantially the same as the amino acid sequence in SEQ ID NO. 44 or 45.

As used herein, G protein-coupled receptor proteins are receptor proteins having a shared structure with seven transmembrane domains, most of which transduce intracellular signals through the activation of coupled guanine nucleotide-binding proteins.

Preferred examples of amino acid sequences represented by SEQ ID NO. 44 include the amino acid sequences represented by SEQ ID NOS. 3, 18, and 32, and especially the amino acid sequence represented by SEQ ID NO. 32.

Preferred examples of amino acid sequences represented by SEQ ID NO. 45 include the amino acid sequences represented by SEQ ID NOS. 6, 21, and 35, and especially the amino acid sequence represented by SEQ ID NO. 35.

Polypeptides of the present invention include polypeptides derived from the cells or tissue (such as the pituitary gland, pancreas, brain, kidney, liver, gonad, thyroid gland, gall bladder, bone marrow, adrenal glands, skin, muscles, lungs, gastrointestinal tract, blood vessels, and heart) of warm-blooded animals (such as humans, guinea pigs, rats, mice, swine, sheep, bovines, and monkeys). Specific examples include those with an amino acid sequence that is the same as or substantially the same as amino acid sequences represented by SEQ ID NO. 44 or 45, and preferably an amino acid sequence that is the same as or substantially the same as the amino acid sequence represented by SEQ ID NO. 3, 18, or 32, or by SEQ ID NO. 6, 21, or 35.

In addition to polypeptides with an amino acid sequence represented by SEQ ID NO. 44 or 45 or preferably an amino acid sequence represented by SEQ ID NO. 3, 18, or 32, or by SEQ ID NO. 6, 21, or 35, other examples of polypeptides in the present invention include polypeptides which have an amino acid sequence with approximately 50 to 99.9% (preferably 70 to 99.9%, more preferably 80 to 99.9%, and even more preferably 90 to 99.9%) homology with the amino acid sequences represented by SEQ ID NO. 44 or 45 and preferably the amino acid sequences represented by SEQ ID NO. 3, 18, and 32, or by SEQ ID NO. 6, 21, and 35, or which have activity substantially the same as polypeptides with an amino acid sequence represented by SEQ ID NO. 44 or 45, and preferably an amino acid sequence represented by SEQ ID NO. 3, 18, or 32, or by SEQ ID NO. 6, 21, or 35. Examples of such activity include the activity of the ligand polypeptides such as receptor binding activity and signal transduction activity, the activity of polypeptides in regulating CRH secretion (such as CRH secretion-promoting activity and CRH secretion-inhibiting activity), and the like. Activity that is "substantially the same" means that characteristics such as receptor binding activity and activity to regulate CRH secretion (such as CRH secretion-promoting activity and CRH secretion-inhibiting activity) are the same. As such, the receptor binding activity may be slightly weaker, and the molecular weight of the ligand polypeptide may vary. Substantially the same peptides derived from humans or the same warm-blooded animals are sometimes found to have differences in the amino acid sequence which are not essential to the peptide, depending on differences in the species from which they are derived, but the polypeptides of the present invention include such peptides with nonessential differences in amino acid sequence.

The ligand polypeptides of the present invention, as well as their production and application, are described in further detail below.

Specific examples of the ligand polypeptides in the present invention include polypeptides derived from rats, bovines, humans, or mice, which have an amino acid sequence represented by SEQ ID NO. 44 or 45 (in SEQ ID NO. 44, Xaa at position 3 is Thr or Ala, Xaa at position 5 is Arg or Gln, Xaa at position 10 is Ile or Thr, Xaa at position 21 is Thr or Ala, and Xaa at position 22 is Gly or Ser; in SEQ ID NO. 45, Xaa at position 10 is Thr or Ala, and Xaa at position 11 is Gly or Ser).

Ligand polypeptides of the present invention also include polypeptides or their amides, esters, or salts, which have:

(i) an amino acid sequence in which between 1 and 15, preferably between 1 and 10, and even more preferably between 1 and 5 amino acids of the amino acid sequence in SEQ ID NO. 44 have been substituted with other amino acids;
(ii) an amino acid sequence in which between 1 and 15, preferably between 1 and 10, and even more preferably between 1 and 5 amino acids of the amino acid sequence in SEQ ID NO. 44 have been deleted;
(iii) an amino acid sequence in which between 1 and 15, preferably between 1 and 10, and even more preferably between 1 and 5 amino acids have been added (inserted) to the amino acid sequence in SEQ ID NO. 44; and
(iv) an amino acid sequence with modifications to constituent amino acids (particularly the side chains) of the polypeptides in (i), (ii), or (iii) above.

Ligand polypeptides of the present invention furthermore include polypeptides or their amides, esters, or salts, which have:

(v) an amino acid sequence in which between 1 and 10, and more preferably between 1 and 5 amino acids of the amino acid sequence in SEQ ID NO. 45 have been substituted with other amino acids;
(vi) an amino acid sequence in which between 1 and 10, and more preferably between 1 and 5 amino acids of the amino acid sequence in SEQ ID NO. 45 have been deleted;
(vii) an amino acid sequence in which between 1 and 10, and more preferably between 1 and 5 amino acids have been added (inserted) to the amino acid sequence in SEQ ID NO. 45; and
(viii) an amino acid sequence with modifications to constituent amino acids (particularly the side chains) of the polypeptides in (v), (vi), or (vii) above.

The substitutions, deletions, additions, modifications, and the like in (i) through (viii) above can be brought about in the amino acid sequence of the ligand polypeptides of the present invention either by design or by chance to allow them to be mutated (transformed) into heat- or protease-stable ligand polypeptides or highly active types of ligand polypeptides in which the physiological activity of the ligand polypeptide is enhanced. Such mutant ligand polypeptides are encompassed in the ligand polypeptides or their amides, esters, or salts in the present invention.

In the present specification, peptides are described according to the usual practice, with the left end as the N terminal (amino terminal) and the right end as the C terminal (carboxyl terminal).

Examples of modifications to the constituent amino acids of the polypeptides in the present invention include the in vivo cleavage of the N-terminal Gln and the conversion of the Gln to pyroglutamate.

In the polypeptides of the present invention, such as polypeptides represented by SEQ ID NO. 44 or 45, the $\alpha$-carboxyl group of the C-terminal amino acid residues is usually a carboxyl group (-COOH) or a carboxylate (-COO), but the carboxyl group of the C-terminal amino acid residues may also be an amide ($-CONH_2$) or an ester (-COOR). R in esters represented by -COOR include $C_1$ to $C_6$ alkyl such as methyl, ethyl, n-propyl, isopropyl, and n-butyl, $C_3$ to $C_8$ cycloalkyl such as cyclopentyl and cyclohexyl, $C_6$ to $C_{12}$ aryl such as phenyl and $\alpha$-naphthyl, and $C_7$ to $C_{14}$ aralkyl such as phenyl $C_1$ to $C_2$ alkyl such as benzyl or phenethyl, diphenyl-$C_1$ to $C_2$ alkyl such as benzhydril,and $\alpha$-naphthyl-$C_1$ to $C_2$ alkyl such as $\alpha$-naphthylmethyl, as well as pivaloyloxymethyl groups and the like which are commonly used as esters for oral purposes.

In cases where the polypeptides of the present invention such as polypeptides represented by SEQ ID NO.

44 or 45 have carboxyl groups or carboxylates other than at the C terminal, polypeptides in which such groups have been converted to amides or esters are included in the polypeptides of the present invention. Esters in such cases are the same as the esters and the like of the C terminal amino acid residues described above.

Especially desirable ligand polypeptides in the present invention are peptides in which carboxyl groups among the C terminal amino acid residues have been converted to amides. In preferred polypeptides, the carboxyl groups among the C terminal amino acid residues of polypeptides with an amino acid sequence represented by SEQ ID NO. 44 or 45, and preferably polypeptides with an amino acid sequence represented by SEQ ID NO. 3, 6, 18, 21, 32, or 35 (especially polypeptides with an amino acid sequence represented by SEQ ID NO. 32 or 35) are converted to amides.

Salts of physiologically acceptable bases (such as alkali metals) or acids (organic or inorganic acids) may be used as the polypeptide salts in the present invention, wtih physiologically acceptable acid salts being preferred. Examples of such salts include salts of inorganic acids (such as hydrochloric acid, phosphoric acid, hydrobromic acid, and sulfuric acid), and salts of organic acids (such as acetic acid, formic acid, propionic acid, fumaric acid, maleic acid, succinic acid, tartaric acid, citric acid, malic acid, oxalic acid, benzoic acid, methanesulfonic acid, and benzenesulfonic acid).

The ligand polypeptides of the present invention can be produced (i) by purifying polypeptides from the cells or tissue of humans or warm-blooded animals or (ii) in accordance with common methods of polypeptide synthesis. They can also be produced (iii) by methods (described below) for culturing transformants that contain DNA coding for such polypeptides.

(i) When the ligand polypeptides are produced from the cells or tissue of humans or warm-blooded animals, the cells or tissue of humans or warm-blooded animals can be homogenized and then extracted with an acid or the like, and the extract can be purified and isolated by a combination of reverse phase chromatography, ion exchange chromatography, affinity chromatography, or the like.

(ii) The ligand peptides can also be produced by a method of polypeptide synthesis that is well-known per se. The peptides can be synthesized by either the solid phase synthesis or liquid phase synthesis, for example. In other words, the target peptide can be produced by the condensation of a partial polypeptide or amino acid capable of forming a ligand polypeptide with the remainder, followed by elimination of any protective groups when the product has protective groups. The methods in (1) through (5) below are examples of commonly known methods of condensation and methods for eliminating protective groups in such cases.

(1) M. Bodanszky and M. A. Ondetti: *Peptide Synthesis,* Interscience Publishers, New York, 1966;
(2) Schroeder and Luebke: *The Peptide,* Academic Press, New York, 1965;
(3) Nobuo Izumiya et al.: *Fundamentals and Experiments in Peptide Synthesis,* Maruzen, 1975;
(4) Haru'aki Yajima and Shunpei Sakakibara: *Biochemical Experiment Series 1*: *Protein Chemistry IV,* 205, 1977; and
(5) Haru'aki Yajima (ed.), Development of Drugs-Continued, 14, Peptide Synthesis, Hirokawa Shoten

The following methods are specific examples of methods for the synthesis of the ligand polypeptides in (ii) above.

A peptide synthesis resin which is suitable for forming amides may be used to synthesize the amide form of such ligand polypeptides. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenylacetamidomethyl resin, polyacrylamide resin, 4-(2',4'-dimethoxyphenyl-hydroxymethyl) phenoxy resin, and 4-(2',4'-dimethoxyphenyl-Fmoc aminoethyl)phenoxy resin. With the use of such resins, amino acids having $\alpha$-amino groups and side chain functional groups protected by common suitable protective groups can be condensed on the resin according to the sequence of the target peptide by well-known condensation methods.

After the reaction, the peptide can be cut out of the resin, and the various protective groups can be removed, giving the target polypeptide.

Various activating reagents which can be used for peptide synthesis can be employed in the condensation of the aforementioned protected amino acids, although carbodiimides are particularly preferred. Examples of such carbodiimides include DCC,
N,N'-diisopropylcarbodiimide, and N-ethyl-N'-(3-dimethylaminopropyl)carbodiimide.

For activation by these, a racemization inhibitor additive (such as HOBt) and the protected amino acids can be added to the resin directly, or the inhibitor can be added in the form of a symmetric acid anhydride, or an HOBt ester or HOOBt ester, after the protected amino acid has been pre-activated. Solvents which can be used for the activation of protected amino acids or their condensation with the resin may be selected from known solvents which can be used in peptide condensation. Examples of such solvents include acid amides such as N,N-dimethyl formamide, N,N-dimethyl acetamide, and N-methyl pyrrolidone, halohydrocarbons such as methylene chloride and chloroform, alcohols such as trifluoroethanol, sulfoxides such as dimethyl sulfoxide, tertiary amines such as pyridine, ethers such as dioxane and tetrahydrofuran, nitriles such as acetonitrile and propionitrile, esters such as methyl acetate and ethyl acetate, or suitable mixtures thereof. The reaction temperature for the condensation can be selected from within the range known to be useful for the formation of peptide bonds, which is usually between about -20 °C to 50°C. The activated amino acid derivative is generally used in a proportion of 1.5- to 4-fold excess. The degree of condensation can be checked using a known ninhydrin reaction. In cases of insufficient condensation, the condensation reaction can be repeated without removing the protective groups until sufficient condensation has been achieved. When repeated condensation fails to result in sufficient condensation, the unreacted amino acids can be acetylated using acetic anhydride or acetylimidazole without affecting subsequent reaction.

Protective groups for the amino groups of starting material amino acids during peptide synthesis include Z, Boc, tert-amyloxycarbonyl, isobornyloxycarbonyl, 4-methoxybenzyloxycarbonyl, Cl-Z, Br-Z, adamantyloxycarbonyl, trifluoroacetyl, phthalyl, formyl, 2-nitrophenylsulfenyl, diphenylphosphinothioyl, and Fmoc. Protective groups for carboxyl groups include the aforementioned $C_1$ to $C_6$ alkyls, $C_3$ to $C_8$ cycloalkyls, and $C_7$ to $C_{14}$ aralkyls, as well as 2-adamantyl, 4-nitrobenzyl, 4-methoxybenzyl, 4-chlorobenzyl, phenacyl and benzyloxycarbonyl hydrazide, tert-butoxycarbonyl hydrazide, and trityl hydrazide.

Hydroxyl groups of serine and threonine can be protected, for example, through esterification or etherification. Groups suitable for such esterification include lower ($C_1$ to $C_6$) alkanoyl groups such as acetyl, aroyl groups such as benzoyl, and carboxylic acid-derived groups such as benzyloxycarbonyl and ethoxycarbonyl. Groups suitable for etherification include benzyl, tetrahydropyranyl, and tert-butyl.

Protective groups for the phenolic hydroxyl group of tyrosine include Bzl, $Cl_2$-Bzl, 2-nitrobenzyl, Br-Z, and tert-butyl.

Examples of protective groups for the imidazole of histidine include Tos, 4-methoxy-2,3,6-trimethylbenzenesulfonyl, DNP, benzyloxymethyl, Bum, Boc, Trt, and Fmoc.

The ligand polypeptides, or their amides or esters, in the present invention can be peptides with any mutation in the amino acid sequence, provided that their activity to regulate CRH secretion, for example, is the same as that of polypeptides with an amino acid sequence which is the same or substantially the same as the amino acid sequence in SEQ ID NO. 44 or 45. Examples of such peptides include peptides with an amino acid sequence in which between 1 and 20 of the amino acids have been deleted from a peptide with the amino acid sequence in SEQ ID NO. 44. Specific desirable examples include: (a) peptides with an amino acid sequence from position 2 to position 31 of the amino acid sequence in SEQ ID NO. 44; (b) peptides with an amino acid sequence from position 3 to position 31 of the amino acid sequence in SEQ ID NO. 44; (c) peptides with an amino acid sequence from position 4 to position 31 of the amino acid sequence in SEQ ID NO. 44; (d) peptides with an amino acid sequence from position 5 to position 31 of the amino acid sequence in SEQ ID NO. 44; (e) peptides with an amino acid sequence from position 6 to position 31 of the amino acid sequence in SEQ ID NO. 44; (f) peptides with an amino acid sequence from position 7 to position 31 of the amino acid sequence in SEQ ID NO. 44; (g) peptides with an amino acid sequence from position 8 to position 31 of the amino acid sequence in SEQ ID NO. 44; (h) peptides with an amino acid sequence from position 9 to position 31 of the amino acid sequence in SEQ ID NO. 44; (i) peptides with an amino acid sequence from position 10 to position 31 of the amino acid sequence in SEQ ID NO. 44; (j) peptides with an amino acid sequence from position 11 to position 31 of the amino acid sequence in SEQ ID NO. 44; (k) peptides with an amino acid sequence from position 12 to position 31 of the amino acid sequence in SEQ ID NO. 44; (1) peptides with an amino acid sequence from position 13 to position 31 of the amino acid sequence in SEQ ID NO. 44; (m) peptides with an amino acid sequence from position 14 to position 31 of the amino acid sequence in SEQ ID NO. 44; (n) peptides with an amino acid sequence from position 15 to position 31 of the amino acid sequence in SEQ ID NO. 44; (o) peptides with an amino acid sequence from position 16 to position 31 of the amino acid sequence in SEQ ID NO. 44; (p) peptides with an amino acid sequence from position 17 to position 31 of the amino acid sequence in SEQ ID NO. 44; (q) peptides with an amino acid sequence from position 18 to position 31 of the amino acid sequence in SEQ ID NO. 44; (r) peptides with an amino acid sequence from position 19 to position 31 of the amino acid sequence in SEQ ID NO. 44; (s) peptides with an amino acid sequence from position 20 to position 31 of the amino acid sequence in SEQ ID NO. 44; and (t) peptides with an amino acid sequence from position 21 to position 31 of the amino acid sequence in SEQ ID NO. 44.

SEQ ID NOS. 3, 18, and 32 which are more preferable examples of amino acid sequences represented by SEQ ID NO. 44 may also have the mutations in amino acid sequence given as examples for the amino acid sequence in SEQ ID NO. 44.

Examples also include peptides with an amino acid sequence in which between 1 and 10 of the amino acids have been deleted from a peptide with the amino acid sequence in SEQ ID NO. 45. Specific desirable examples include: (a) peptides with an amino acid sequence from position 2 to position 20 of the amino acid sequence in SEQ ID NO. 45; (b) peptides with an amino acid sequence from position 3 to position 20 of the amino acid sequence in SEQ ID NO. 45; (c) peptides with an amino acid sequence from position 4 to position 20 of the amino acid sequence in SEQ ID NO. 45; (d) peptides with an amino acid sequence from position 5 to position 20 of the amino acid sequence in SEQ ID NO. 45; (e) peptides with an amino acid sequence from position 6 to position 20 of the amino acid sequence in SEQ ID NO. 45; (f) peptides with an amino acid sequence from position 7 to position 20 of the amino acid sequence in SEQ ID NO. 45; (g) peptides with an amino acid sequence from position 8 to position 20 of the amino acid sequence in SEQ ID NO. 45; (h) peptides with an amino acid sequence from position 9 to position 20 of the amino acid sequence in SEQ ID NO. 45; (i) peptides with an amino acid sequence from position 10 to position 20 of the amino acid sequence in SEQ ID NO. 45; and (j) peptides with an amino acid sequence from position 11 to position 20 of the amino acid sequence in SEQ ID NO. 45.

The ligand polypeptides of the present invention also include whole rat brain-derived or bovine hypothalamic-derived peptides with the amino acid sequences in SEQ ID NOS. 1 and 15. These polypeptides may also have the mutations in amino acid sequence given as examples for the amino acid sequences in SEQ ID NOS. 44 and 45. These polypeptides are also examples of precursors of polypeptides having the amino acid sequences in SEQ ID NOS. 44 and 45.

Other examples of ligand polypeptides of the present invention in addition to the above include polypeptides with the amino acid sequences in SEQ ID NOS. 4, 5, 7, 8, 19, 20, 22, 23, 33, 34, 36, and 37. These polypeptides, too, may have the mutations in amino acid sequence given as examples for the amino acid sequences in SEQ ID NO. 45.

SEQ ID NOS. 6, 21, and 35 which are more preferable examples of amino acid sequences represented by SEQ ID NO. 45 may also have the mutations in amino acid sequence given as examples for the amino acid sequence in SEQ ID NO. 45.

The ligand polypeptides of the present invention may also be in the form of fusion proteins with other proteins (such as common proteins with well-known functions or characteristics).

DNA coding for the ligand polypeptides of the present invention may be any having a base sequence that codes for a polypeptide with an amino acid sequence that is the same or substantially the same as the amino acid sequences in SEQ ID NOS. 44 or 45 of the present invention. Examples include genomic DNA, genomic DNA libraries, tissue and cell cDNA, tissue and cell cDNA libraries, and synthetic DNA. Vectors used in libraries may be any from among bacteriophages, plasmids, cosmids, phagemids, and the like. RNA fractions prepared from tissue and cells can also be used directly for amplification by RT-PCR (reverse transcription PCR).

More specifically, DNA with the base sequence in SEQ ID NO. 2 or the like can be used as DNA coding for rat whole brain-derived or bovine hypothalamic-derived polypeptides having the amino acid sequences in SEQ ID NOS. 1 and 15.

The R at position 129 in SEQ ID NO. 2 is G or A, and the Y at positions 179 and 240 are C or T. When the Y at position 179 is C, the amino acid sequence in SEQ ID NO. 1 is encoded, and when the Y at position 179 is T, the amino acid sequence in SEQ ID NO. 15 is encoded.

DNA having a base sequence represented by SEQ ID NO. 9, 10, 11, 12, 13, or 14 can be used as DNA coding for bovine-derived polypeptides with the amino acid sequences in SEQ ID NOS. 3, 4, 5, 6, 7, and 8, respectively. The R at position 63 in SEQ ID NOS. 9, 10, and 11, and the R at position 30 in SEQ ID NOS. 12, 13, and 14 are G or A.

DNA having a base sequence represented by SEQ ID NO. 17, 24, 25, 26, 27, 28, or 29 can be used as DNA coding for rat polypeptides with the amino acid sequences in SEQ ID NOS. 16, 18, 19, 20, 21, 22, and 23, respectively.

DNA having a base sequence represented by SEQ ID NO. 31, 38, 39, 40, 41, 42, or 43 can be used as DNA coding for human polypeptides with the amino acid sequences in SEQ ID NOS. 30, 32, 33, 34, 35, 36, and 37, respectively.

DNA fragments containing a partial base sequence of between 6 and 90 bases, for example (preferably between 6 and 60, more preferably between 9 and 30, and still more preferably between 12 and 30) from among the DNA coding for bovine polypeptides with an amino acid sequence represented by SEQ ID NO. 1 or 15, rat polypeptides with an amino acid sequence represented by SEQ ID NO. 16, or human polypeptides with an amino acid sequence represented by SEQ ID NO. 30, can preferably be used as probes to find DNA.

(iii) The polypeptides in the present invention can be produced as described below by culturing transformants containing DNA coding for such polypeptides.

DNA fully coding for the polypeptides of the present invention can be cloned in the following manner. Specifically, (1) DNA having a partial base sequence for such a polypeptide can be synthesized for use as primers in PCR amplification of DNA fully coding for the polypeptide, or (2) a DNA library obtained upon the incorporation of cDNA or genomic DNA, or DNA fragments thereof, into suitable vectors can be screened by hybridization with labeled synthetic DNA or DNA fragments containing some or all regions of the ligand polypeptide, for example. Hybridization should be managed in accordance with the methods given, for example, in *Molecular Cloning* (2nd Ed.: J. Sambrook et al., Cold Spring Harbor Lab. Press (1989)). When a commercially available DNA library is used, the method given in the accompanying protocol should be followed.

The DNA base sequence can be transformed by methods that are well-known per se or methods based on them, such as ODA-LA PCR, the gapped duplex method, or the Kunkel method, using PCR or a common kit such as Mutan™-Super Express Km (Takara Shuzo) or Mutan™-K (Takara Shuzo).

The cloned DNA coding for the polypeptide can be used as such or after being digested with the desired restriction enzymes or after the addition of linker DNA. The DNA may have ATG as the translation initiation codon on the 5' terminal side, and TAA, TGA, or TAG as the translation termination codon on the 3' terminal side. The translation initiation and termination codons can be added using suitable synthetic DNA adapters

Expression vectors which contain DNA with a base sequence coding for such a polypeptide can be prepared, for example, by (a) cutting out the target DNA fragment from DNA containing the DNA coding for a polypeptide of the present invention, and (b) ligating the DNA fragment downstream of the promoter sequence in a common suitable expression vector.

Examples of vectors which can be used include *E. coli* plasmids (such as pBR322, pBR325, pUC12, and pUC13), Bacillus *subtilis* plasmids (such as pUB110, pTP5, and pC194), yeast plasmids (such as pSH19 and pSH15), bacteriophages such as λ phages, and animal viruses such as retroviruses, vaccinia viruses, and baculoviruses. Promoters include any capable of functioning in the host that is used to express the gene coding for the target polypeptide.

Promoters that are preferred for *E. coli* hosts during transformation include trp promoters, lac promoters, recA promoters, λPL promoters, and lpp promoters; examples that are preferred for Bacillus hosts include SPO1 promoters, SPO2 promoters, and penP promoters; and examples that are preferred for yeast hosts include PHO5 promoters, PGK promoters, GAP promoters, and ADH promoters. Examples that are preferred for animals hosts include SV40-derived promoters, retrovirus promoters, metallothionein promoters, heat shock promoters, cytomegalovirus (CMV) promoters, and SRα promoters. An enhancer should be used to ensure efficient expression of the gene coding for the target polypeptide.

A signal sequence compatible with the host may also be added to the N-terminal side of the polypeptide as needed. Alalkaline phosphatase signal sequences, OmpA signal sequences, and the like can be used for E. *coli* hosts; α-amylase signal sequences, subtilisin signal sequences, and the like can be used for Bacillus hosts; mating factor-α signal sequences, invertase signal sequences, and the like can be used for yeast hosts; and insulin signal sequences, α-interferon signal sequences, antibody molecule signal sequences, and the like can be used for animal cell hosts. Transformants can be produced using vectors that contain DNA coding for polypeptides, which have been constructed in the manner described above.

Examples of hosts for use in transformation include Escherichia microorganisms, Bacillus genus microorganisms, yeasts, insect cells, and animal cells.

Specific examples of Escherichia microorganisms include *Escherichia coli* K12·DH1 (*Proc. Natl. Acad. Sci. USA,* Vol. 60, p. 160 (1968)), JM103 (*Nucleic Acids Research,* Vol. 9, p. 309 (1981)), JA221 (*Journal of Molecular Biology,* Vol. 120, p. 517 (1978)), HB101 (*Journal of Molecular Biology,* Vol. 41, p. 459 (1969)), and C600 (*Genetics,* Vol. 39, p. 440 (1954)).

Examples of Bacillus microorganisms include *Bacillus subtilis* MI114 *(Gene,* Vol. 24, p. 255 (1983)) and 207-21 (*Journal of Biochemistry,* Vol. 95, p. 87 (1984)).

Examples of yeasts include *Saccharomyces cerevisiae* AH22, AH22R, NA87-11A, DKD-5D, and 20B-12.

Examples of insects include silkworm larvae (Maeda et al, *Nature,* Vol. 315, p. 592 (1985)).

Examples of animal cells include monkey cells COS-7, Vero, Chinese hamster ovary cells CHO, DHFR gene-deficient Chinese hamster cells CHO (dhfr CHO cells), mouse L cells, mouse myeloma cells, and human FL cells.

Escherichia microorganisms can be transformed in accordance with methods as disclosed in, for example, *Proc. Natl. Acad. Sci. USA,* Vol. 69, p. 2110 (1972), and *Gene,* Vol. 17, p. 107 (1982).

Bacillus microorganisms can be transformed in accordance with methods as disclosed in, for example, *Molecular & General Genetics,* Vol. 168, p. 111 (1979).

Yeasts can be transformed in accordance with methods as disclosed in, for example, *Proc. Natl. Acad. Sci. USA,* Vol. 75, p. 1929 (1978).

Insect cells can be transformed in accordance with methods as disclosed in, for example, *Bio/Technology*, Vol. 6, p. 47 (1988).

Animal cells can be transformed by methods as disclosed in, for example, *Virology,* Vol. 52, p. 456 (1973).

In this manner it is possible to obtain transformants which have been transformed in expression vectors that contain DNA coding for polypeptides.

Liquid media are preferred for the culture of transformants obtained with Escherichia or Bacillus hosts, and should be prepared with the carbon sources, nitrogen sources, minerals, and the like which are needed for the growth of the transformants. Examples of carbon sources include glucose, dextrin, soluble starch, and sucrose. Examples of nitrogen sources include organic or inorganic substances such as ammonium salts, nitrates, corn steep liquor, peptone, casein, meat extract, soybean cake, and potato extract. Examples of minerals include calcium chloride, sodium dihydrogen phosphate, and magnesium chloride. Yeast extract, vitamins, growth-promoting factors, and the like may also be added to the medium as needed. The medium pH may be any pH permitting the growth of the transformants, but is usually about 5 to 8.

The preferred medium for culturing Escherichia microorganisms is M9 medium containing, for example, glucose and casamino acid (Miller, *Journal of Experiments in Molecular Genetics,* p. 431, Cold Spring Harbor Laboratory, New York (1972)). The medium may be supplemented as needed with drugs such as 3β-indolyl acrylic acid in order to ensure promoter efficiency. Escherichia hosts can usually be cultured for about 3 to 24 hours at about 15 to 43°C.

Bacillus hosts can usually be cultured while aerated or stirred as needed for about 6 to 24 hours at about 30 to 40°C.

Examples of media for the culture of transformants using yeast hosts include Burkholder minimum medium (K.L. Bostian et al., *Proc. Natl. Acad. Sci. USA,* Vol. 77, p. 4505 (1980)), and SD medium containing 0.5% casamino acid (G.A. Bitter et al., *Proc. Natl. Acad. Sci. USA,* Vol. 81, p. 5330 (1984)).

The medium pH should be adjusted to between about 5 and 8. Culture usually lasts about 24 to 72 hours at about 20 to 35°C, while aerated and stirred as needed.

Examples of media for the culture of transformants using insect hosts include Grace's insect medium (T.C.C. Grace, Nature, Vol. 195, p. 788 (1962)) suitably supplemented with an additive such as 10% inactivated bovine serum. The medium pH should be adjusted to between about 6.2 and 6.4. Culture usually lasts about 3 to 5 days at about 27°C, while aerated and stirred as needed.

Examples of media for the culture of transformants using animal cell hosts include MEM medium containing about 5 to 20% fetal bovine serum *(Science,* Vol. 122, p. 501 (1952)), DMEM medium *(Virology,* Vol. 8, p. 396 (1959)), RPMI 1640 medium *(Journal of the American Medical Association,* Vol. 199, p. 519 (1967)), and 199 medium (Proceedings of the Society for the Biological Medicines, Vol. 73, 1 (1950)). The pH should be between about 6 and 8. Culture usually lasts about 15 to 60 hours at about 30 to 40°C, while aerated and stirred as needed.

The polypeptides should be isolated and purified from the aforementioned culture (culture broth and cultured bacterial cells or cultured cells) by means of the following methods, for example.

When the polypeptides are allowed to accumulate in cultured bacterial cells or cultured cells, the bacterial cells or cells are collected in the usual manner after the culture and are suspended in a suitable buffer, the cells are then disrupted by commonultrasonic treatment, lysozyme treatment, and/or lyophilization or the like, and the target polypeptides are then obtained in the form of extract by common centrifugation, filtration, or the like. The buffer can be supplemented as needed with protein denaturants such as urea or guanidine hydrochloride, or surfactant such as Trion X-100 (Wako Pure Chemicals, registered trademark: henceforth abbreviated as TM).

When the polypeptides are secreted in the culture, the cultured bacterial cells or cultured cells are separated in the usual manner from the supernatant, giving the polypeptides in the form of culture supernatant.

The polypeptides contained in the resulting culture supernatant or extract can be purified by a suitable combination of isolation and purification methods that are well-known per se. Examples of such methods include: (1) methods exploiting solubility, such as salting out or solvent precipitation; (2) methods primarily exploiting differences in molecular weight, such as dialysis, ultrafiltration, gel filtration and SDS-polyacrylamide gel electrophoresis; (3) methods exploiting differences in electrical charge, such as ion-exchange chromatography; (4) methods exploiting specific affinity, such as affinity chromatography; (5) methods exploiting differences in hydrophobicity, such as reverse-phase high-performance liquid chromatography; and (6) methods exploiting differences in isoelectric point, such as isoelectric focusing or chromatofocusing.

Polypeptides obtained in free form can be converted to a salt by a method that is well-known per se, or a method based thereon. Conversely, polypeptides obtained in the form of salts can be converted to free form or to another salt by a method that is well-known per se, or a method based thereon.

Either before or after the purification of the polypeptide, a suitable protein-modifying enzyme can be allowed to act on the peptide in the usual manner to add any modifications to the polypeptide or to remove portions of the sequence. Examples of such enzymes include trypsin, chymotrypsin, arginyl endopeptidase, protein kinase, and glycosidase. The activity of the resulting mutant polypeptide can be assayed by testing the receptor binding or by means of enzyme immunoassay using specific antibodies.

The ligand polypeptides of the present invention have activity to regulate CRH secretion, that is, CRH secretion-promoting and inhibiting activity. Specifically the ligand polypeptides of the present invention have activity to promote CRH secretion, as the test examples below will reveal, and can thus be used as prophylactics and remedies (medicinal drugs or animal drugs) for a variety of diseases related to CRH under-secretion. Because of their potent receptor protein affinity, the ligand polypeptides of the present invention also have activity to inhibite CRH secretion as a result of desensitization of CRH secretion when given in higher dosages. In such cases, the ligand polypeptides can be used for the treatment and prevention of various diseases related to CRH over-secretion.

As such, the ligand polypeptides of the present invention can be useful as CRH secretion promoters for the relief, prevention, and treatment of various diseases related to CRH secretion, such as hypoaldosteronism, hypocortisolemia, secondary and chronic adrenocorticism, Addison's disease (weakness, nausea, pigmentation, hypogonadism, alopecia, and hypotension), adrenal dysfunction, and obesity.

The ligand polypeptides of the present invention can also be useful as CRH secretion inhibitors for the relief, prevention, and treatment of various diseases related to CRH secretion, such as Cushing's disease, Cushing's syndrome (central obesity, moon face, hypertension, purplish pink stretch marks, and hirsutism), adrenocorticotrophic hormone (ACTH)-producing pituitary tumors, CRH-producing tumors, aldosertone-producing tumors, aldosteronism (primary and secondary), primary cortisol resistance, congenital adrenal enzyme deficiency, high stress, depression, anorexia nervosa, insomnia, stomach and duodenal ulcers, and irritable bowel syndrome.

The ligand polypeptides of the present invention are useful as test agents for investigating CRH secretion function.

The polypeptides of the present invention can be prepared in the usual manner for use as medicinal drugs or animal drugs. For example, they can be used orally in the form of optionally sugar-coated tablets, capsules, elixirs, microcapsules or the like, they can be used parenterally in the form of injections such as sterile solutions or suspensions with water or other pharmaceutically acceptable liquids, or they can be used in the form of nasal drops. These preparations can be produced, for example, by mixing the polypeptide or salt thereof with physiologically acceptable carriers, flavoring agents, excipients, vehicles, antiseptics, stabilizers, binders, or the like, in the unit dose forms required in generally accepted pharmaceutical manufacturing. The content of the active ingredient in these preparations should give the appropriate dose within the specified range.

Examples of additives which can be mixed with tablets, capsules, and the like include binders such as gelatin, corn starch, tragacanth, and gum arabic; excipients such as crystalline cellulose; extenders such as corn starch, gelatin, and alginic acid; lubricants such as magnesium stearate; sweetening agents such as sucrose, lactose, and saccharin; and flavoring agents such as peppermint, akamono oil, and cherry. In the case of capsule unit dose forms, the aforementioned types of materials can also include liquid carriers such as oils and fats. Sterile compositions for injection can be formulated by ordinary pharmaceutical manufacturing methods such as the dissolution or suspension of active ingredients and naturally occurring vegetable oils such as sesame oil and coconut oil in a vehicle such as water for injection.

Aqueous liquids for injection include physiological saline and isotonic solutions containing glucose and other adjuvants (such as D-sorbitol, D-mannitol, and sodium chloride), and may be used in combination with appropriate dissolution aids such as alcohols (such as ethanol), polyalcohols (such as propylene glycol and polyethylene glycol), and nonionic surfactants (such as Polysorbate 80(™) and HCO-50). Oleaginous liquids include sesame oil and soybean oil, and may be used in combination with dissolution aids such as benzyl benzoate and benzyl alcohol. The above may also be blended with buffers (such as phosphate buffer and sodium acetate buffer), soothing agents (such as benzalkonium chloride and procaine hydrochloride), stabilizers (such as human serum albumin and polyethylene glycol), preservatives (such as benzyl alcohol and phenol), antioxidants, and the like. Suitable ampules are usually aseptically filled with the resulting injection liquid.

Because the resulting preparation is safe and has low toxicity, it can be administered, for example, to mammals (such as humans, mice, rats, guinea pigs, rabbits, sheep, swine, bovines, felines, canines, monkeys, and chimpanzees) .

Although the dosage of CRH secretion regulators containing a polypeptide of the present invention will vary depending on the symptoms or the like, the oral dosage for patients with hypoaldosteronism may generally range from about 0.1 to 100 mg, preferably from about 1.0 to 50 mg, and even more preferably from about 1.0 to 20 mg at a time, in terms of the polypeptide of the present invention (per 60 kg body weight). The single parenteral dose will vary depending on the purpose of administration, symptoms, method of administration, and the like, but in the form of an injection, for example, the dosage for patients with hypoaldosteronism may usually be about 0.01 to about 30 mg, preferably about 0.1 to about 20 mg, and even more preferably about 0.1 to about 10 mg at a time, in terms of the polypeptide of the present invention (per 60 kg body weight), given by intravenous injection. Doses for animals can be given as calculated per 60 kg body weight.

The G protein-coupled receptor proteins (hereinafter sometimes referred to simply as receptor proteins) or ligand polypeptides used in the present invention can be prepared, for example, in accordance with the descriptions in WO96/0530 and WO97/24436.

Specific receptor proteins include proteins, or their salts, which have an amino acid sequence that is the same or substantially the same as the amino acid sequence in SEQ ID NO. 46. An amino acid sequence that is "substantially the same" means an amino acid sequence that may have mutations, provided that the protein activity such as receptor-ligand (ligand polypeptide of the present invention) binding activity is substantially the same (is not significantly changed), which is the same meaning as for the receptor proteins defined in WO96/05302 and WO97/24436.

Amino acid sequences such as the one represented in SEQ ID NO. 48 are specific examples of amino acid sequences that are substantially the same as the amino acid sequence represented in SEQ ID NO. 46.

Examples of base sequences of DNA coding for the amino acid sequence represented in SEQ ID NO. 46 include those such as the base sequence represented in SEQ ID NO. 47, and examples of base sequences of DNA coding for the amino acid sequence represented in SEQ ID NO. 48 include those such as the base sequence represented in SEQ ID NO. 49.

The following descriptions concern the production and application of CRH secretion regulators comprising compounds or their salts for modifying binding between receptor proteins and the ligand polypeptides of the present invention for such receptor proteins.

Compounds or their salts for modifying binding between receptor proteins and the ligand polypeptides of the present invention for such receptor proteins include compounds or their salts that promote the functions (such as CRH secretion activity) of the ligand polypeptides of the present invention, as well as compounds or their salts that inhibit the functions (such as CRH secretion activity) of the ligand polypeptides of the present invention.

Because the ligand polypeptides of the present invention have action in regulating CRH secretion and the like (such as CRH secretion-promoting activity and such as CRH secretion-inhibiting activity), compound or their salts that promote the functions (such as CRH secretion activity) of the ligand polypeptides of the present invention can be useful as CRH secretion regulators for the relief, prevention, and treatment of various diseases related to CRH secretion, such as hypoaldosteronism, hypocortisolemia, secondary and chronic adrenocorticism, Addison's disease (weakness, nausea, pigmentation, hypogonadism, alopecia, and hypotension), adrenal dysfunction, and obesity, and also as analgesics.

Compounds or their salts that inhibit the CRH secretion activity of the ligand polypeptides of the present invention can also be useful for the relief, prevention, and treatment of various diseases related to CRH secretion, such as Cushing's disease, Cushing's syndrome (central obesity, moon face, hypertension, purplish pink stretch marks, and hirsutism), adrenocorticotrophic hormone (ACTH)-producing pituitary tumors, CRH-producing tumors, aldosterone-producing tumors, aldosteronism (primary and secondary), primary cortisol resistance, congenital adrenal enzyme deficiency, high stress, depression, anorexia nervosa, insomnia, stomach and duodenal ulcers, and irritable bowel syndrome.

The ligand polypeptides of the present invention may therefore be useful as reagents for screening compounds or their salts that promote or inhibit the functions (such as CRH secretion activity) of the ligand polypeptides of the present invention.

Compounds or their salts that promote or inhibit the functions of the ligand polypeptides of the present invention can be obtained, for example, by screening compounds that affect binding between G protein-coupled receptor proteins (such as phGR3, and UHR-1 (WO96/05302 and WO97/24436)) and the ligand polypeptides of the present invention.

Screening methods are detailed below.

A G protein-coupled receptor protein (such as phGR3, and UHR-1 (WO96/05302 and WO97/24436)) expression system can be constructed, and a receptor binding assay system using the expression system can be employed for efficient screening of compounds (such as peptides, proteins, non-peptide compounds, synthetic compounds, and fermented products) or their salts which modify binding between the ligand polypeptides of the present invention and G protein-coupled receptor proteins (such as phGR3, and UHR-1 (WO96/05302 and WO97/24436)).

Such compounds include (a) compounds with G protein-coupled receptor-mediated cell-stimulating activity (such as activity in promoting or inhibiting arachidonic acid release, acetylcholine release, intracellular $Ca^{2+}$ release, intracellular cAMP production, intracellular cGMP production, inositol phosphate production, changes in cell membrane potential, intracellular protein phosphorylation, c-fos activation, and decreases in pH), (b) compounds with no cell-stimulating activity (referred to as receptor protein antagonists), and (c) compounds which attenuate the binding power between the ligand polypeptides of the present invention and G protein-coupled receptor proteins.

Specifically, compounds or their salts which modify binding between receptor proteins and the ligand polypeptides of the present invention for such receptor proteins can be obtained by screening compounds or their salts which modify binding between the ligand polypeptides of the present invention and receptor proteins, characterized by comparing (i) cases in which a receptor protein and a ligand polypeptide of the present invention are brought into contact with each other and (ii) cases in which a test compound is brought into contact with a receptor protein and a ligand polypeptide of the present invention.

Such a screening method is characterized, for example, by comparing assays of the extent to which ligands bind to receptor proteins, the cell-stimulating activity, or the like between cases (i) and (ii).

Specific examples of such a screening method include:

(i) methods for screening compounds or their salts which modify binding between the ligand polypeptides of the present invention and receptor proteins of the present invention, characterized by comparative assay of the extent to which labeled ligand polypeptides of the present invention bind to receptor proteins between cases in which labeled ligand polypeptides of the present invention are brought into contact with receptor proteins of the present invention, and cases in which labeled ligand polypeptides of the present invention and test compounds are brought into contact with receptor proteins of the present invention;

(ii) methods for screening compounds or their salts which modify binding between the ligand polypeptides of the present invention and receptor proteins of the present invention, characterized by comparative assay of the extent to which labeled ligand polypeptides of the present invention bind to fractions between cases in which labeled ligand polypeptides of the present invention are brought into contact with cells or cell membrane fractions containing the receptor proteins of the present invention, and cases in which labeled ligand polypeptides of the present invention and test compounds are brought into contact with cells or cell membrane fractions containing the receptor proteins of the present invention; and

(iii) methods for screening compounds or their salts which modify binding between the ligand polypeptides of the present invention and receptor proteins of the present invention, characterized by comparative assay of the extent to which labeled ligand polypeptides of the present invention bind to fractions between cases in which labeled ligand polypeptides of the present invention are brought into contact with receptor proteins expressed on cell membranes through the culture of transformants containing DNA coding for the receptor proteins of the present invention, and cases in which labeled ligand polypeptides of the present invention and test compounds are brought into contact with receptor proteins expressed on cell membranes through the culture of transformants containing DNA coding for the receptor proteins of the present invention.

The aforementioned screening methods are described in greater detail below.

Examples of receptor proteins of the present invention which can be used in the aforementioned screening methods include any containing the aforementioned receptor proteins of the present invention, although cell membrane fractions of mammalian organs containing receptor proteins are preferred. However, because of the extreme difficulties involved in procuring human organs in particular, human receptor proteins which have been expressed in large amounts using recombinants are suitable for use in screening.

The receptor proteins of the present invention are preferably produced by the animal cell or insect cell expression of DNA coding for the receptor proteins. Although cDNA is usually used for DNA fragments coding for portions of the target protein, the invention is by no means limited thereto. Gene fragments or synthetic DNA may also be used, for example. To ensure efficient expression of DNA fragments coding for a receptor protein after being introduced into an animal cell host, such DNA fragments should be incorporated downstream of a polyhedrin promoter from a nuclear polyhedrosis virus (NPV) belonging to an insect-host baculovirus, an SV40 promoter, a retrovirus promoter, a metallothionein promoter, a human heat shock promoter, a cytomegalovirus promoter, or an SR$\alpha$ promoter. The quantity and quality of the expressed receptors can be checked by methods that are well-known per se. This can be done according to a method in the literature (such as P. Nambi et al, *J. Biol. Chem.*, Vol. 267, pp. 19555-19559 (1992)).

As such, examples of products containing the receptor proteins of the present invention in the aforementioned screening methods may include receptor proteins which have been purified by methods that are well-known per se, as well as cells containing receptor proteins or cell membrane fractions containing receptor proteins.

When cells containing receptor proteins of the present invention are used in the aforementioned screening methods, the cells may be immobilized with glutaraldehyde, formalin, or the like. The cells can be immobilized in accordance with a method that is well-known per se.

Cells containing receptor proteins of the present invention refer to host cells in which such receptor proteins are expressed. Desirable examples of such host cells include *Escherichia coli, Bacillus subtilis,* yeasts, insect cells, and animal cells.

Cell membrane fractions refer to fractions containing an abundance of cell membranes, which are obtained by methods that are well-known per se after the cells have been disrupted. Methods for disrupting cells include methods for crushing cells with a Potter-Elvehjem homogenizer, disruption with a Waring blender or a Polytron (manufactured by Kinematica), ultrasonic disruption, and disruption using a French press or the like, where the cells are discharged under pressure through narrow nozzles. Fractions of cell membranes are obtained primarily through fractionation with centrifugal force, such as fraction centrifugation or density gradient centrifugation. For example, cell lysates are centrifuged for a short period of time (usually about 1 to 10 minutes) at low speed (500 to 3,000 rpm), and the supernatant is then usually further centrifuged for 30 minutes to 2 hours at high speed (150,000 to 300,000 rpm), giving membrane fractions in the form of precipitate. Such membrane fractions contain an abundance of the expressed receptor proteins of the present invention and membrane components such as cell phospholipids or membrane proteins.

The amount of the receptor protein contained in the cells or membrane fractions which contain such receptor proteins should be $10^3$ to $10^8$ molecules, and more preferably $10^5$ to $10^7$ molecules, per cell. The greater the amount expressed, the higher the ligand binding activity (specific activity) per membrane fraction, which not only allows the construction of highly sensitive screening systems but also allows the assay of large amounts of sample per lot.

Suitable receptor protein fractions and labeled ligand polypeptides of the present invention, for example, can be used to screen for compounds that modify binding between ligand polypeptides of the present invention and receptor proteins of the present invention.

Preferred examples of receptor protein fractions include native receptor protein fractions, and recombinant receptor protein fractions with equivalent activity. As used herein, "equivalent activity" means equivalent ligand binding activity, signal transducing activity, and the like.

Labeled ligands include labeled ligands and labeled ligand analog compounds. Examples include ligands labeled with [$^3H$], [$^{125}I$], [$^{14}C$], and [$^{35}S$] .

In order to screen compounds that modify binding between the ligand polypeptides of the present invention and receptor proteins of the present invention, a receptor protein preparation can first be prepared by suspending cells or cell membrane fractions containing receptor proteins of the present invention in buffer suitable for screening. Examples of buffer include any that will not inhibit binding between ligands and receptor proteins, such as Tris-HCl buffer or phosphate buffer with a pH of 4 to 10 (and preferably a pH of 6 to 8). Surfactant such as CHAPS, Tween-80™ (by Kao-Atlas), digitonin, and deoxycholate can be added to the buffer to reduce non-specific binding. A protease inhibitor such as PMSF, leupeptin, E-64 (manufactured by Peptide Institute, Inc.), or pepstatin can also be added to inhibit the degradation of ligands or receptors by proteases. A certain amount (5,000 cpm to 500,000 cpm) of labeled ligand is added to 0.01 to 10 ml of the above receptor solution in the presence of $10^{-4}$ M to $10^{-10}$ M test compound. A reaction tube with an excess of unlabeled ligand is also prepared to determine the non-specific binding (NSB). The reaction is carried out for about 20 minutes to 24 hours, and preferably about 30 minutes to 3 hours, at a temperature of about 0°C to 50°C, and preferably about 4°C to 37°C. After the reaction, the reaction mixture is filtered through glass fiber filter paper or the like and is washed with a suitable amount of the same buffer, and the radioactivity remaining on the glass fiber filter paper is measured with a liquid scintillation counter or γ-counter. Candidates with antagonist-inhibiting capacity, that is, compounds or salts that inhibit the function of ligand polypeptides of the present invention (such as CRH secreting activity), can be selected from test compounds with a specific binding (B-NSB) of no more than 50%, for example, where 100% is the count ($B_0$-NSB) calculated by subtracting the non-specific binding (NSB) from the count prevailing in the absence of any competing substances ($B_0$).

To implement the method for screening compounds that modify binding between the ligand polypeptides of the present invention and receptor proteins of the present invention, for example, the receptor protein-mediated cell-stimulating activity (such as activity in promoting or inhibiting arachidonic acid release, acetylcholine release, intracellular $Ca^{2+}$ release, intracellular cAMP production, intracellular cGMP production, inositol phosphate production, changes in cell membrane potential, intracellular protein phosphorylation, c-fos activation, and decreases in pH) can be assayed by a common method or with a commercially available assay kit.

Specifically, cells containing receptor proteins of the present invention are first cultured in multi-well plates or the like. In performing the screening, the medium is replaced with fresh medium or a suitable buffer that is not toxic to the cells, test compound or the like is added, the mixture is incubated for a certain period of time, the cells are then extracted or the supernatant is collected, and the product is assayed according to a variety of methods. When the production of a substance serving as an indicator of cell-stimulating activity (such as arachidonic acid) proves difficult to detect because of degrading enzymes in the cells, the assay may be performed with the addition of an inhibitor for the enzyme. Activity such as the inhibition of cAMP production can be detected in terms of the inhibited production of cells whose basic production is increased with forskolin or the like.

Cells expressing suitable receptor proteins may be used for screening by assay of cell-stimulating activity. Preferred cells for expressing receptor proteins of the present invention include cell lines with native receptor proteins of the present invention and cells expressing the aforementioned recombinant receptor proteins.

Examples of test compounds include peptide, proteins, non-peptide compounds, synthetic compounds, fermented products, cell extracts, plant extracts, and animal tissue extracts. Such compounds may be novel compounds or well-known compounds.

Screening kits for compounds or their salts that modify binding between ligand polypeptides of the present invention and receptor proteins of the present invention can include receptor proteins of the present invention, cells containing receptor proteins of the present invention, or cell membrane fractions containing receptor proteins of the present invention.

The following are examples of such a screening kit.

1. Screening reagents

(i) Assay buffer and washing buffer

Hanks' Balanced Salt Solution (by Gibco) supplemented with 0.05% bovine serum albumin (by Sigma).

This can be sterilized by filtration with a filter having a pore size of 0.45 μm, and stored at 4°C, or it can be prepared at the time of use.

(ii) G protein-coupled receptor preparation

CHO cells expressing receptor proteins of the present invention, which have been subcultured at $5 \times 10^5$ cells/well in 12-well plates, and cultured for 2 days at 37°C in 5% $CO_2$ and 95% air

(iii) Labeled ligands

Ligand peptides of the present invention labeled with commercially available [$^3$H], [$^{125}$I], [$^{14}$C], [$^{35}$S] or the like
Stored in the form of aqueous solution at 4°C or-20°C, and diluted with assay buffer to 1 μM at the time of use

(iv) Ligand label solution

The ligand polypeptide of the present invention is dissolved to a concentration of 1 mM in PBS containing 0.1% bovine serum albumin (by Sigma), and stored at -20°C.

2. Assay

(i) CHO cells expressing the receptor proteins of the present invention, which have been cultured in 12-well tissue culture plates, are washed twice with 1 ml assay buffer, and 490 μl assay buffer is added per well.
(ii) 5 μl of $10^{-3}$ to $10^{-10}$ M test compound solution is added to the plates, 5 μl labeled ligand is then added, and a reaction is conducted for about 1 hour at ambient temperature. 5 μl of $10^{-3}$ M ligand polypeptide is added instead of test compound to determine the non-specific binding.
(iii) The reaction solution is removed, and the cells are washed 3 times with 1 ml washing buffer. The labeled ligand binding to the cells is dissolved in 0.2 N NaOH-1% SDS and mixed with 4 ml liquid Scintillator A (Wako Pure Chemicals).
(iv) The radioactivity is assayed using a liquid scintillation counter (Beckman), and the Percent Maximum Binding (PMB) is determined using the following equation.

$$PMB = [(B-NSB)/(B_0-NSB)] \times 100$$

PMB: Percent Maximum Binding
B: value when sample added
NSB: non-specific binding
$B_0$: maximum binding

Common procedures can be followed when using compounds of salts thereof that modify the binding between the ligand polypeptides of the present invention for the receptor proteins of the present invention and the receptor proteins, as CRH secretion regulators as described above. For example, they can be used orally in the form of optionally sugar-coated tablets, capsules, elixirs, microcapsules or the like, they can be used parenterally in the form of injections such as sterile solutions or suspensions with water or other pharmaceutically acceptable liquids, or they can be used in the form of nasal drops. These preparations can be produced, for example, by mixing the compound or salt thereof with physiologically acceptable carriers, flavoring agents, excipients, vehicles, antiseptics, stabilizers, binders, or the like, in the unit dose forms required in generally accepted pharmaceutical manufacturing. The content of the active ingredient in these preparations should give the appropriate dose within the specified range.
Examples of additives which can be mixed with tablets, capsules, and the like include binders such as gelatin, corn starch, tragacanth, and gum arabic; excipients such as crystalline cellulose; extenders such as corn starch, gelatin, and alginic acid; lubricants such as magnesium stearate; sweetening agents such as sucrose, lactose, and saccharin; and flavoring agents such as peppermint, akamono oil, and cherry. In the case of capsule unit dose forms, the aforementioned types of materials can also include liquid carriers such as oils and fats. Sterile compositions for injection can be formulated by ordinary pharmaceutical manufacturing methods such as the dissolution or suspension of active ingredients and naturally occurring vegetable oils such as sesame oil and coconut oil in a vehicle such as water for injection.
Aqueous liquids for injection include physiological saline and isotonic solutions containing glucose and other adjuvants (such as D-sorbitol, D-mannitol, and sodium chloride), and may be used in combination with appropriate dissolution aids such as alcohols (such as ethanol), polyalcohols (such as propylene glycol and polyethylene glycol), and nonionic surfactants (such as Polysorbate 80(™) and HCO-50). Oleaginous liquids include sesame oil and soybean oil, and may be used in combination with dissolution aids such as benzyl benzoate and benzyl alcohol. The above may also be blended with buffers (such as phosphate buffer and sodium acetate buffer), soothing agents (such as benzalkonium chloride and procaine hydrochloride), stabilizers (such as human serum albumin and polyethylene glycol), preservatives (such as benzyl alcohol and phenol), antioxidants, and the like. Suitable ampules are usually aseptically filled with the resulting injection liquid.

Because the resulting preparation is safe and has low toxicity, it can be administered, for example, to mammals (such as humans, mice, rats, guinea pigs, rabbits, sheep, swine, bovines, felines, canines, monkeys, and chimpanzees).

Although the dosage of CRH secretion regulators containing a compound or salt that modifies binding between ligand polypeptides for receptor proteins of the present invention and receptor proteins of the present invention will vary depending on the symptoms or the like, the oral dosage for patients with hypoaldosteronism may generally range from about 0.1 to 100 mg, preferably from about 1.0 to 50 mg, and even more preferably from about 1.0 to 20 mg at a time, in terms of the compound or salt (per 60 kg body weight). The single parenteral dose will vary depending on the purpose of administration, symptoms, method of administration, and the like, but in the form of an injection, for example, the dosage for patients with hypoaldosteronism may usually be about 0.01 to about 30 mg, preferably about 0.1 to about 20 mg, and even more preferably about 0.1 to about 10 mg at a time, in terms of the polypeptide of the present invention (per 60 kg body weight), given by intravenous injection. Doses for other animals can be given as calculated per 60 kg body weight.

The SEQ ID NOS. in the Sequence Listing of the present Specification indicate the following sequences.

SEQ ID NO. 1

Full-length base sequence of bovine hypothalamic ligand peptide

SEQ ID NO. 2

Full-length base sequence of bovine hypothalamic ligand peptide cDNA

SEQ ID NO. 3

Amino acid sequence of bovine hypothalamic ligand peptide, corresponding to the amino acid sequence from position 23 to position 53 in SEQ ID NO. 1

SEQ ID NO. 4

Amino acid sequence of bovine hypothalamic ligand peptide, corresponding to the amino acid sequence from position 23 to position 54 in SEQ ID NO. 1

SEQ ID NO. 5

Amino acid sequence of bovine hypothalamic ligand peptide, corresponding to the amino acid sequence from position 23 to position 55 in SEQ ID NO. 1

SEQ ID NO. 6

Amino acid sequence of bovine hypothalamic ligand peptide, corresponding to the amino acid sequence from position 34 to position 53 in SEQ ID NO. 1

SEQ ID NO. 7

Amino acid sequence of bovine hypothalamic ligand peptide, corresponding to the amino acid sequence from position 34 to position 54 in SEQ ID NO. 1

SEQ ID NO. 8

Amino acid sequence of bovine hypothalamic ligand peptide, corresponding to the amino acid sequence from position 34 to position 55 in SEQ ID NO. 1

SEQ ID NO. 9

Base sequence of DNA coding for bovine hypothalamic ligand polypeptide (SEQ ID NO. 3)

SEQ ID NO. 10

Base sequence of DNA coding for bovine hypothalamic ligand polypeptide (SEQ ID NO. 4)

SEQ ID NO. 11

Base sequence of DNA coding for bovine hypothalamic ligand polypeptide (SEQ ID NO. 5)

SEQ ID NO. 12

Base sequence of DNA coding for bovine hypothalamic ligand polypeptide (SEQ ID NO. 6)

SEQ ID NO. 13

Base sequence of DNA coding for bovine hypothalamic ligand polypeptide (SEQ ID NO. 7)

SEQ ID NO. 14

Base sequence of DNA coding for bovine hypothalamic ligand polypeptide (SEQ ID NO. 8)

SEQ ID NO. 15

Full-length amino acid sequence for ligand polypeptide found in genomic DNA

SEQ ID NO. 16

Full-length amino acid sequence for rat ligand polypeptide

SEQ ID NO. 17

Full-length base sequence for rat ligand polypeptide cDNA

SEQ ID NO. 18

Amino acid sequence for rat ligand polypeptide, corresponding to the amino acid sequence from position 22 to position 52 in SEQ ID NO. 16

SEQ ID NO. 19

Amino acid sequence for rat ligand polypeptide, corresponding to the amino acid sequence from position 22 to position 53 in SEQ ID NO. 16

SEQ ID NO. 20

Amino acid sequence for rat ligand polypeptide, corresponding to the amino acid sequence from position 22 to position 54 in SEQ ID NO. 16

SEQ ID NO. 21

Amino acid sequence for rat ligand polypeptide, corresponding to the amino acid sequence from position 33 to position 52 in SEQ ID NO. 16

SEQ ID NO. 22

Amino acid sequence for rat ligand polypeptide, corresponding to the amino acid sequence from position 33 to position 53 in SEQ ID NO. 16

SEQ ID NO. 23

Amino acid sequence for rat ligand polypeptide, corresponding to the amino acid sequence from position 33 to position 54 in SEQ ID NO. 16

SEQ ID NO. 24

Base sequence of DNA coding for rat ligand polypeptide (SEQ ID NO. 18)

SEQ ID NO. 25

Base sequence of DNA coding for rat ligand polypeptide (SEQ ID NO. 19)

SEQ ID NO. 26

Base sequence of DNA coding for rat ligand polypeptide (SEQ ID NO. 20)

SEQ ID NO. 27

Base sequence of DNA coding for rat ligand polypeptide (SEQ ID NO. 21)

SEQ ID NO. 28

Base sequence of DNA coding for rat ligand polypeptide (SEQ ID NO. 22)

SEQ ID NO. 29

Base sequence of DNA coding for rat ligand polypeptide (SEQ ID NO. 23)

SEQ ID NO. 30

Full-length amino acid sequence for human ligand polypeptide

SEQ ID NO. 31

Full-length base sequence of human ligand polypeptide cDNA

SEQ ID NO. 32

Amino acid sequence of human ligand polypeptide, corresponding to the amino acid sequence from position 23 to position 53 in SEQ ID NO. 30

SEQ ID NO. 33

Amino acid sequence of human ligand polypeptide, corresponding to the amino acid sequence from position 23 to position 54 in SEQ ID NO. 30

SEQ ID NO. 34

Amino acid sequence of human ligand polypeptide, corresponding to the amino acid sequence from position 23 to position 55 in SEQ ID NO. 30

SEQ ID NO. 35

Amino acid sequence of human ligand polypeptide, corresponding to the amino acid sequence from position 34 to position 53 in SEQ ID NO. 30

SEQ ID NO. 36

Amino acid sequence of human ligand polypeptide, corresponding to the amino acid sequence from position 34 to position 54 in SEQ ID NO. 30

SEQ ID NO. 37

Amino acid sequence of human ligand polypeptide, corresponding to the amino acid sequence from position 34 to position 55 in SEQ ID NO. 30

SEQ ID NO. 38

Base sequence of DNA coding for human ligand polypeptide, (SEQ ID NO. 32)

SEQ ID NO. 39

Base sequence of DNA coding for human ligand polypeptide, (SEQ ID NO. 33)

SEQ ID NO. 40

Base sequence of DNA coding for human ligand polypeptide, (SEQ ID NO. 34)

SEQ ID NO. 41

Base sequence of DNA coding for human ligand polypeptide, (SEQ ID NO. 35)

SEQ ID NO. 42

Base sequence of DNA coding for human ligand polypeptide, (SEQ ID NO. 36)

SEQ ID NO. 43

Base sequence of DNA coding for human ligand polypeptide, (SEQ ID NO. 37)

SEQ ID NO. 44

Amino acid sequence for ligand polypeptides of the present invention, where Xaa at position 3 is Thr or Ala, Xaa at position 5 is Arg or Gln, Xaa at position 10 is Ile or Thr, Xaa at position 21 is Thr or Ala, and Xaa at position 22 is Gly or Ser

SEQ ID NO. 45

Amino acid sequence for ligand polypeptides of the present invention, where Xaa at position 10 is Thr or Ala, and Xaa at position 11 is Gly or Ser

SEQ ID NO. 46

Amino acid sequence for phGR3, a receptor of ligand polypeptides in the present invention

SEQ ID NO. 47

Base sequence of DNA coding for amino acid sequence for phGR3, a receptor of ligand polypeptides in the present invention

SEQ ID NO. 48

Amino acid sequence for UHR-1, a receptor of ligand polypeptides in the present invention

SEQ ID NO. 49

Base sequence of DNA coding for amino acid sequence for UHR-1, a receptor of ligand polypeptides in the present invention

The *Escherichia coli* JM109/pHGR3 transformant with the base sequence coding for the amino acid sequence of phGR3 in SEQ ID NO. 47 was registered with the Accession No. FERM BP-4807 at the National Institute of Bioscience and Human Technology of the Agency of Industrial Science and Technology in the Ministry of International Trade and Industry, 1-1-3 Higashi, Tsukuba-shi, Ibaraki-ken, Japan on September 27, 1994, and with the Accession No. IFO 15748 on September 22, 1994 at the Institute for Fermentation, Osaka (IFO) at 2-17-85 Juso-honmachi, Yodogawa-ku, Osaka, Japan.

Examples

The present invention is illustrated in further detail in the following examples, but the scope of the present invention is not limited by these examples.

Example 1: Effect on concentrations of adrenocorticotrophic hormone (ACTH) and β-endorphins in blood after intraventricular administration (third ventricle) of PrRP-31 (peptide consisting of the amino acid sequence in SEQ ID NO. 32, in which the carboxyl group of the C terminal amino acid residues of the peptide is an amide)

Adult male Wistar rats (body weight 350 to 380 mg at surgery) were anesthetized with intraperitoneal administration of 50 mg/kg pentobarbital and immobilized in stereotaxic instruments. The incisor bar was set 3.3 mm below the interaural line. The skull was exposed, and a hole was drilled with a dental drill to implant a guide cannula AG-12 (inside diameter of 0.4 mm, outside diameter of 0.5 mm, by Eicom) in the third ventricle. Anchor screws were implanted in four surrounding locations. The tip of the stainless steel guide cannula AG-12 was inserted into position in the upper part of the third ventricle. The stereotaxic coordinates were AP: +7.1 mm, L: 0.0 mm, H: +2.0 mm, from the interaural line, according to the atlas of Paxinos and Watson (1986). The guide cannula was secured to the skull with instant adhesive, dental cement, and the anchor screws. A stainless-steel dummy cannula AD-12 (0.35 mm outside diameter, by Eicom) was then inserted into the guide cannula and secured with a cap nut (by Eicom). The rats were post-operatively housed in individual cages.

The animals were allowed to recover for about 1 week after the implantation of the guide cannulas, and blood samples were taken as they were allowed to move freely. The rats were then anesthetized with intraperitoneal administration of 50 mg/kg pentobarbital. They were then immobilized in the dorsal position on dissection pads, and the left jugular vein was exposed. Polyethylene tubes SP35 (inside diameter 0.5 mm, outside diameter 0.9 mm, by Natsume Seisakusho) were cut to a length of about 30 cm, filled with physiological saline containing 200 units/ml heparin, inserted about 4.5 cm into the jugular vein, and secured. The other end of the tube was passed under the dorsal skin and exposed at the neck (dorsal region).

One night following surgery, 400 μl blood was drawn using a 1 ml syringe and 25-gage needle (both by Terumo) 30 minutes prior to the administration of PrRP-31. To prevent the blood from coagulating, the syringe was filled with 20 μl physiological saline containing 200 units/ml heparin. The cap nut and dummy cannula were then removed from the rat skulls, and a stainless steel microinjection cannula (inside diameter of 0.17 mm, outside diameter of 0.35 mm, by Eicom) connected to a Teflon tube (50 cm long, inside diameter of 0.1 mm, outside diameter of 0.35 mm, by Eicom) was inserted instead. The tip of the microinjection cannula was adjusted so that the tip protruded 1 mm from the guide cannula. One end of the Teflon tube was connected to a microsyringe pump, and a total of 10 μl of either phosphate-buffered saline containing 0.5% bovine serum albumin (BSA) or phosphate-buffered saline containing 0.5% BSA with PrRP-31 (1, 3, or 10 nmol) dissolved therein was injected to the lateral ventricle at a flow rate of 5 μl/min. 15 minutes after the injection, the microinjection cannula was removed, and the dummy cannula was again secured with the cap nut. 700 μl blood samples were drawn via the jugular vein 15 minutes before the start of intraventricular administration and 0, 5, 15, 30, 45, and 60 minutes after. The drawn blood was centrifuged (5,000 rpm, 10 min) using a high speed refrigerated microcentrifuger (MR-150, by Tomy Seiko), and the supernatant (plasma) was collected. The ACTH and β-endorphins contained in the plasma were assayed by radioimmunoassay (ACTH by Mitsubishi Kagaku, β-endorphins by Peninsula). Figures 1 and 2 show that the groups given PrRP-31 had higher concentration-dependent increases in the ACTH and β-endorphin concentrations in blood, peaking at 15 minutes after dosing, compared to the control group.

Example 2: Activity of PrRP-31 in Inhibiting Increases in ACTH and β-endorphin Concentrations in Blood With CRH Antibodies

Four hundred (400) μg of either anti-CRH IgG (by Phoenix Pharmaceutical) or normal rabbit IgG (by Pepro Tech EC) was dissolved in phosphate-buffered saline containing 0.5% bovine serum albumin (BSA) for administration via the jugular vein to adult male Wistar rats prepared in the same manner as in Example 1, and 10 nmol of PrRP-31 was given by intraventricular administration 30 minutes later. 700 μl blood samples were drawn via the jugular vein 0, 5, 15, 30, 45, and 60 minutes after the start of intraventricular administration, the drawn blood was centrifuged (5,000 rpm, 10 min) using a high speed refrigerated microcentrifuger (MR-150, by Tomy Seiko), and the supernatant (plasma) was collected. The ACTH and β-endorphins contained in the plasma were assayed by radioimmunoassay (ACTH by Mitsubishi Kagaku, β-endorphins by Peninsula). Figures 3 and 4 reveal that the groups given anti-CRH IgG showed less of an increase in the ACTH and β-endorphin concentrations in blood as a result of PrRP-31 administration compared to the group given normal rabbit IgG.

Example 3: Effect of Stress on In Vivo PrRP-31

In view of the fact that stress loading results in the secretion of PrRP-31 from cells that produce PrRP-31 or nerve endings containing PrRP-31, which causes elevated in vivo concentrations of PrRP-31, the inventors assayed PrRP-31 concentrations in the cerebrospinal fluid during water-immersion stress loading. Male Wistar rats (body weight 200 to 220 g) were placed in stress cages (W 265 × L 95 × H 200 mm, by Natsume Seisakusho) and were immersed in water (25°C) up to the neck for water-immersion stress loading. Rats were taken out of the water 0, 0.5, 1, 2, 4, 6, and 8 hours after starting, and were anesthetized with intraperitoneal administration of 50 mg/kg pentobarbital. Samples of cerebrospinal fluid were drawn according to the cisternal puncture method of R.B. Chou et al. (*J. Pharmacol. Exp. Ther.,* 219:42-48 (1981)). Specifically, incisions were made in the scalp, with the animals in the prone position, and an excision was made in the muscle layer. A winged needle (25G, by Terumo) attached to a 1 ml syringe (by Terumo) was inserted approximately 2 mm from the base of the skull (mid-dorsal side), and samples were drawn into the syringe when the tip of the needle had been inserted about 5 mm. Upon the insertion of the needle, transparent cerebrospinal fluid flowed into the syringe. After the flow of cerebrospinal fluid was confirmed, the needle was secured with instant adhesive, and samples of cerebrospinal fluid were taken for about 10 minutes. Sampling was stopped when blood appeared in the tube, the tube was clamped with hemostatic forceps, and the cerebrospinal fluid was introduced into Eppendorf tubes containing 2 mM EDTA, 300 KIU/ml aprotinin (by Boehringer Mannheim-Yamanouchi), and CHAPS (by Wako Pure Chemicals) in a final concentration of 0.05%. The collected cerebrospinal fluid was stored frozen, and was centrifuged four times at 13,000 rpm × 5 min to collect the supernatant, which was frozen. The concentration of PrRP in the cerebrospinal fluid was assayed with a high-sensitivity assay system (WO99/60112) prepared using two types of monoclonal antibodies specific to PrRP-31 with different recognition sites (prepared by the inventors). The concentration of PrRP-31 (mean ± SD) in the cerebrospinal fluid was 2.90 ± 0.25 fmol/ml and 2.60 ± 0.25 fmol/ml at 0.5 and 1 hour, respectively, after water-immersion stress loading, which were significantly higher ($P < 0.01$, n=7) than the control concentration of 0.92 ± 0.10 fmol/ml, and continued to decrease over time, as shown in Figure 5. Radioimmunoassay (Mitsubishi Kagaku) of the ACTH concentration in blood during water-immersion stress loading revealed that the concentrations decreased over time after peaking at 0.5 hour, as shown in Figure 6, indicating the same profile as the PrRP-31 concentration in the cerebrospinal fluid.

Example 4: Activity of PrRP-31 in Inhibiting Increases in ACTH Concentration in Blood With CRH Antagonist

Two (2) mg of the CRH antagonist α-helical CRF (by Peninsula Laboratories Europe) dissolved in 0.5 ml physiological saline was administered via the jugular vein to adult male Wistar rats prepared in the same manner as in Example 1. 0.5 ml physiological saline was administered via the jugular vein to the control group. After 15 minutes, 10 nmol of PrRP-31 was given by intraventricular administration to both groups. 400 μl blood samples were drawn via the jugular vein 0, 10, 20, 30, 45, and 60 minutes after the start of intraventricular administration, the drawn blood was centrifuged (5,000 rpm, 10 min) using a high speed refrigerated microcentrifuger (MR-150, by Tomy Seiko), and the supernatant (plasma) was collected. The ACTH in the plasma was assayed by radioimmunoassay (ACTH by Mitsubishi Kagaku). Figure 7 reveals significant inhibition of increases in the ACTH concentration in blood as a result of intraventricular administration of PrRP-31 in groups given the α-helical CRF compared to the physiological saline control group.

Preparation Example 1

Fifty (50) mg ligand polypeptide of the present invention is dissolved in 50 ml of Japan Pharmacopoeia distilled water for injection, and Japan Pharmacopoeia distilled water for injection is then added to bring the total to 100 ml. The solution is then filtered under aseptic conditions, and injection vials are then filled with 1 ml solution apiece under aseptic conditions and freeze-dried.

Preparation Example 2

100 mg ligand polypeptide of the present invention is dissolved in 50 ml of Japan Pharmacopoeia distilled water for injection, and Japan Pharmacopoeia distilled water for injection is then added to bring the total to 100 ml. The solution is then filtered under aseptic conditions, and injection vials are then filled with 1 ml solution apiece under aseptic conditions and freeze-dried.

## INDUSTRIAL APPLICABILITY

The ligand polypeptides of the present invention have activity to regulate CRH secretion, that is, activity to promote and inhibit CRH secretion. In other words, the ligand polypeptides of the present invention have activity to promote CRH secretion and can thus be used in the prevention and treatment of various diseases related to CRH secretion insufficiency. Because of their potent receptor protein affinity, the ligand polypeptides of the present invention also have activity to inhibite CRH secretion as a result of desensitization of CRH secretion when given in higher dosages. In such cases, the ligand polypeptides can be used for the treatment and prevention of various diseases related to CRH over-secretion.

As such, the ligand polypeptides of the present invention can be useful as CRH secretion promoters for the relief, prevention, and treatment of various diseases related to CRH secretion, such as hypoaldosteronism, hypocortisolemia, secondary and chronic adrenocorticism, Addison's disease (weakness, nausea, pigmentation, hypogonadism, alopecia, and hypotension), adrenal dysfunction, and obesity.

The ligand polypeptides of the present invention can also be useful as CRH secretion inhibitors for the relief, prevention, and treatment of various diseases related to CRH secretion, such as Cushing's disease, Cushing's syndrome (central obesity, moon face, hypertension, purplish pink stretch marks, and hirsutism), adrenocorticotrophic hormone (ACTH)-producing pituitary tumors, CRH-producing tumors, aldosertone-producing tumors, aldosteronism (primary and secondary), primary cortisol resistance, congenital adrenal enzyme deficiency, high stress, depression, anorexia nervosa, insomnia, stomach and duodenal ulcers, and irritable bowel syndrome.

The ligand polypeptides of the present invention are useful as test agents for investigating CRH secretion function.

## Claims

1. A corticotropin-releasing hormone secretion regulator, comprising a ligand peptide for a G protein-coupled receptor protein, or an amide thereof, ester thereof, or salt thereof.

2. A corticotropin-releasing hormone secretion regulator according to Claim 1, wherein the ligand peptide for a G protein-coupled receptor protein, or amide thereof, ester thereof, or salt thereof comprises a polypeptide or amide thereof, ester thereof, or salt thereof comprising an amino acid sequence that is the same as or substantially the same as the amino acid sequence represented by SEQ ID NO. 44 or 45.

3. A corticotropin-releasing hormone secretion regulator according to Claim 2, wherein the amino acid sequence represented by SEQ ID NO. 44 is the amino acid sequence represented by SEQ ID NO. 3, 18 or 32.

4. A corticotropin-releasing hormone secretion regulator according to Claim 2, wherein the amino acid sequence represented by SEQ ID NO. 45 is the amino acid sequence represented by SEQ ID NO. 6, 21 or 35.

5. A corticotropin-releasing hormone secretion regulator according to Claim 1, wherein the G protein-coupled receptor protein comprises a protein comprising an amino acid sequence that is the same as or substantially the same as the amino acid sequence represented by SEQ ID NO. 46.

6. A corticotropin-releasing hormone secretion regulator according to Claim 1, which is a corticotropin-releasing hormone secretion promoter.

7. A corticotropin-releasing hormone secretion regulator according to Claim 1, which is a pharmaceutical agent for the treatment or prevention of hypoaldosteronism, hypocortisolemia, secondary or chronic hypoadrenocorticism, Addison's disease, adrenal dysfunction, or obesity.

**8.** A corticotropin-releasing hormone secretion regulator, comprising a compound, or salt thereof, which alters binding between a G protein-coupled receptor protein and the ligand peptide for said G protein-coupled receptor protein.

**9.** The use of a ligand peptide for a G protein-coupled receptor protein, or an amide thereof, ester thereof, or salt thereof for the production of drugs having activity to regulate secretion of corticotropin-releasing hormones.

**10.** A method for regulating the secretion of corticotropin-releasing hormones, **characterized by** administering a ligand peptide for a G protein-coupled receptor protein, or an amide thereof, ester thereof, or salt thereof to mammals.

Fig.1

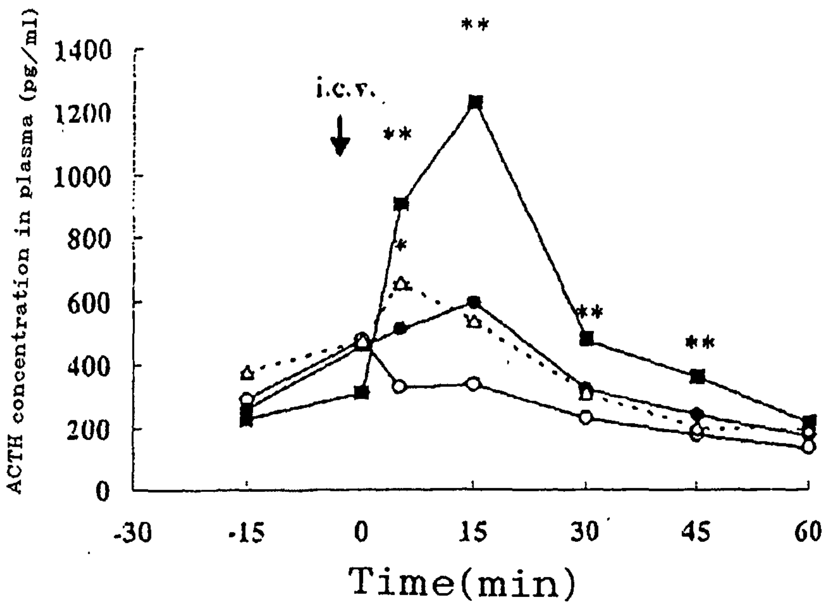
ACTH concentration in plasma (pg/ml)
1400
1200
1000
800
600
400
200
0
i.c.v.
**
**
*
**
**
-30
-15
0
15
30
45
60
Time(min)

Fig.2

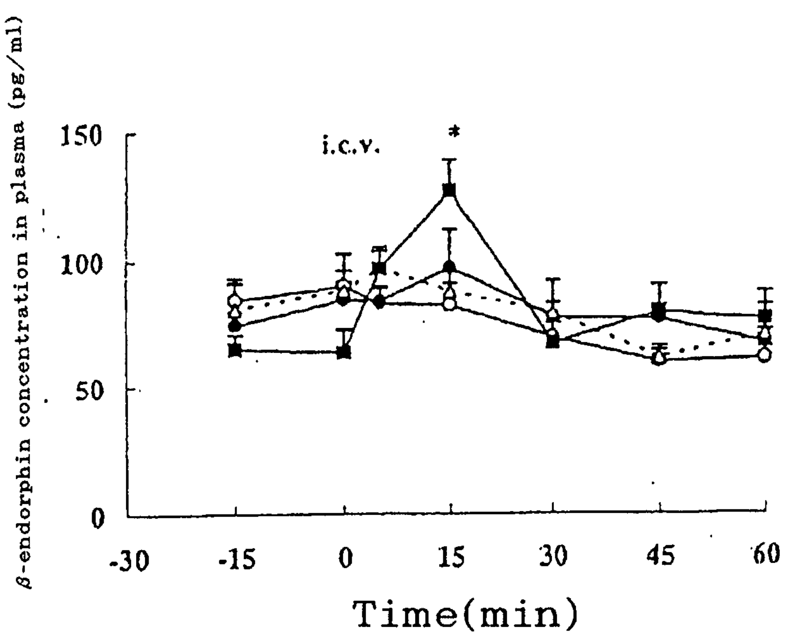
β-endorphin concentration in plasma (pg/ml)
150
100
50
0
i.c.v.
*
-30
-15
0
15
30
45
60
Time(min)

Fig.3

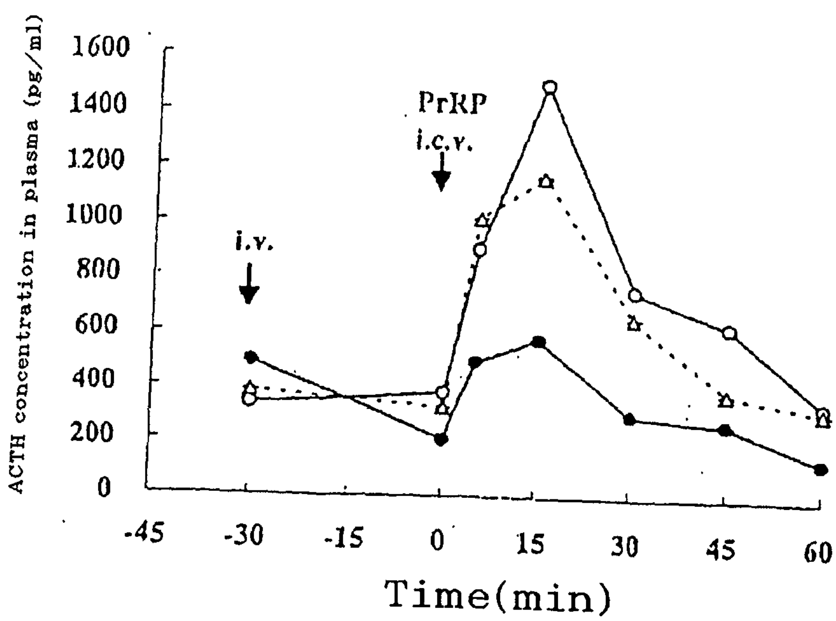
ACTH concentration in plasma (pg/ml)
1600
1400
1200
1000
800
600
400
200
0
PrRP
i.c.v.
i.v.
-45
-30
-15
0
15
30
45
60
Time(min)

Fig.4

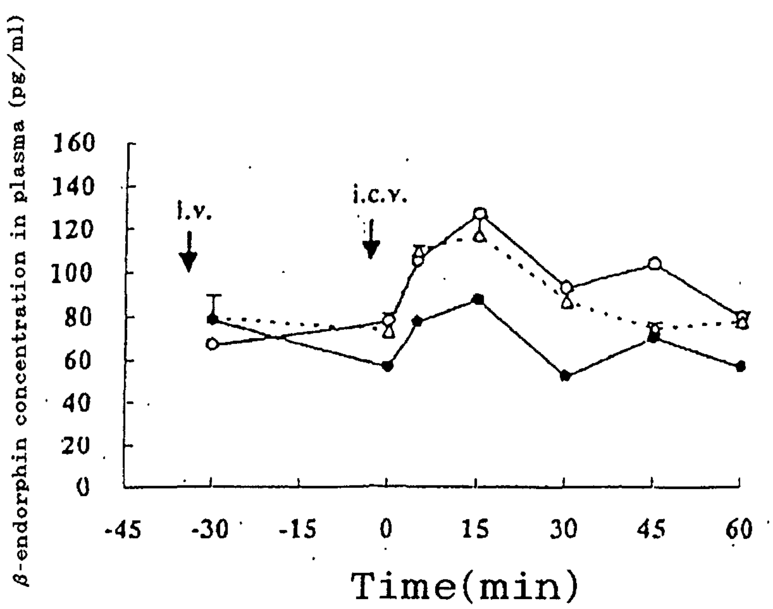
β-endorphin concentration in plasma (pg/ml)
160
140
120
100
80
60
40
20
0
i.v.
i.c.v.
-45
-30
-15
0
15
30
45
60
Time(min)

Fig.5

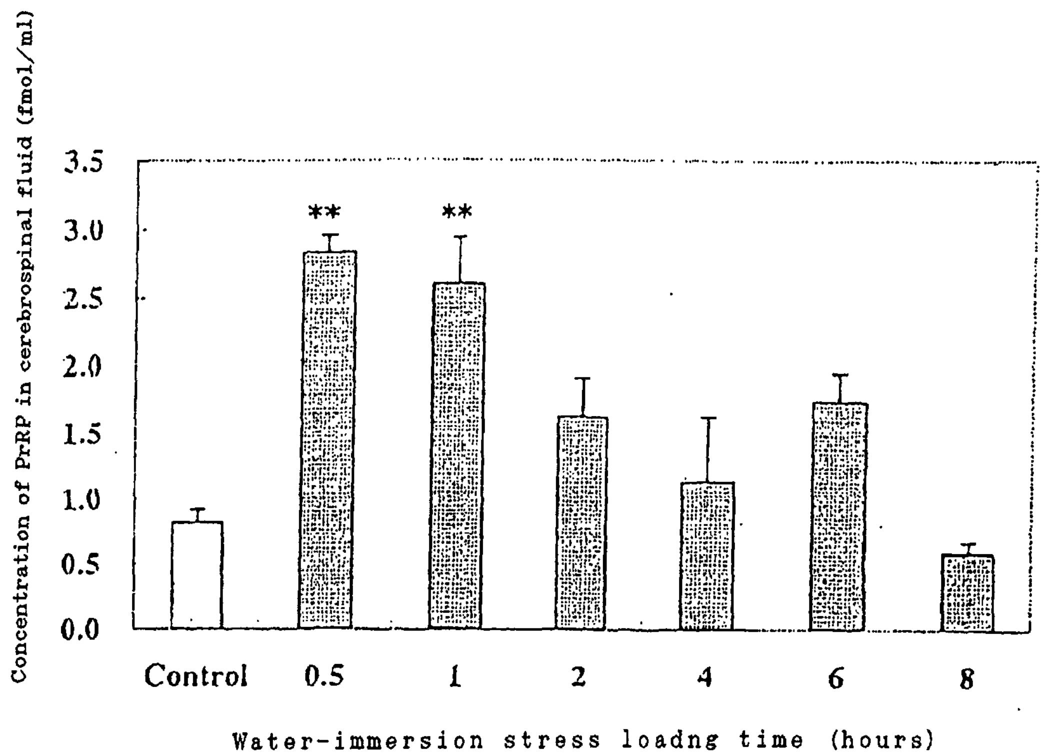
Concentration of PrRP in cerebrospinal fluid (fmol/ml)
3.5
3.0
2.5
2.0
1.5
1.0
0.5
0.0
**
**
Control
0.5
1
2
4
6
8
Water-immersion stress loadng time (hours)

Fig.6

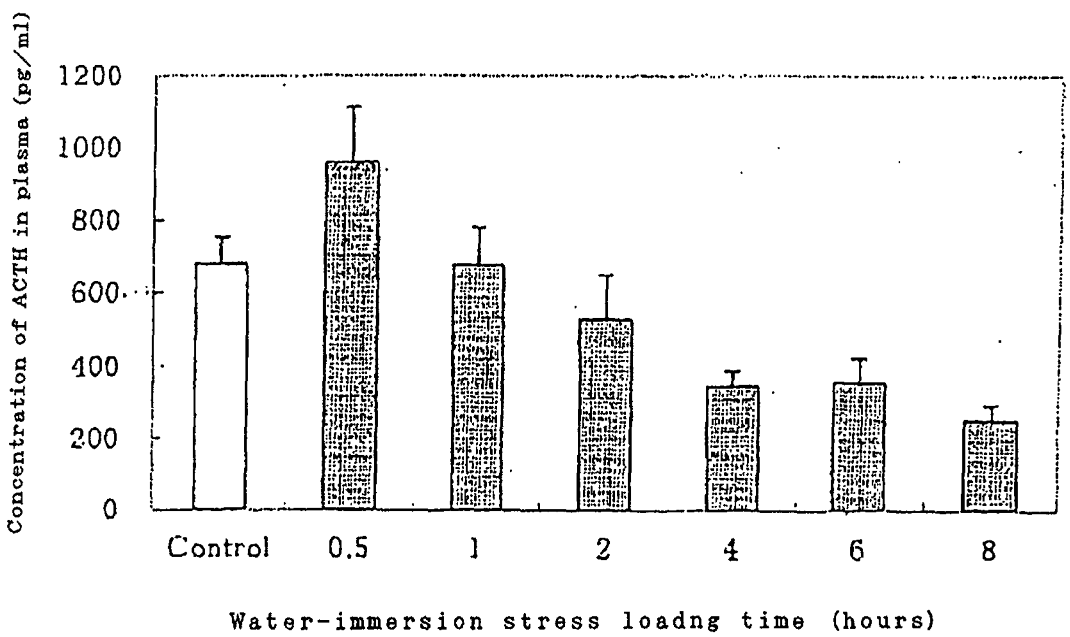
Concentration of ACTH in plasma (pg/ml)
1200
1000
800
600
400
200
0
Control
0.5
1
2
4
6
8
Water-immersion stress loadng time (hours)

Fig.7

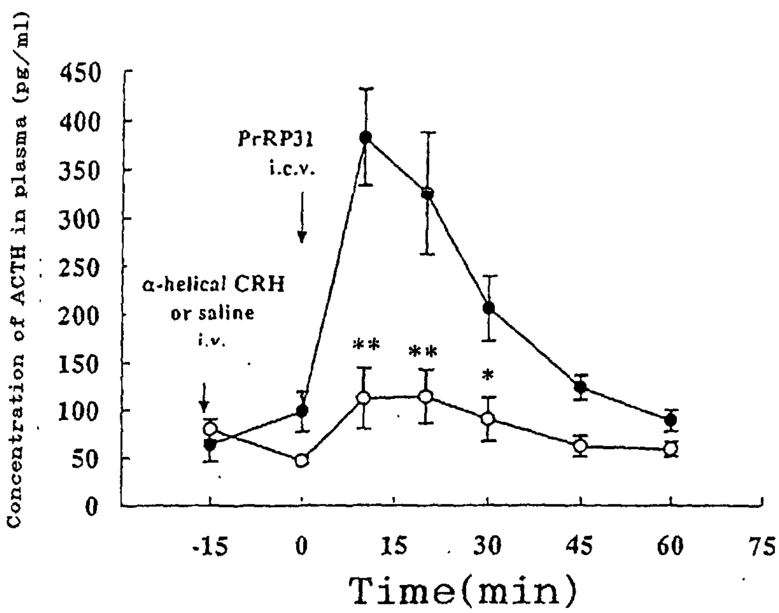
Concentration of ACTH in plasma (pg/ml)
450
400
350
300
250
200
150
100
50
0
PrRP31
i.c.v.
α-helical CRH
or saline
i.v.
**
**
*
-15
0
15
30
45
60
75
Time(min)

# INTERNATIONAL SEARCH REPORT

International application No.
PCT/JP00/08119

A. CLASSIFICATION OF SUBJECT MATTER
Int.Cl$^7$ A61K38/17, A61P5/40, A61P3/04

According to International Patent Classification (IPC) or to both national classification and IPC

B. FIELDS SEARCHED

Minimum documentation searched (classification system followed by classification symbols)
Int.Cl$^7$ A61K38/17, A61P5/40, A61P3/04

Documentation searched other than minimum documentation to the extent that such documents are included in the fields searched

Electronic data base consulted during the international search (name of data base and, where practicable, search terms used)
CAPLUS (STN), MEDLINE (STN), BIOSIS (STN), Genbank/EMBL/DDBJ/GeneSeq

C. DOCUMENTS CONSIDERED TO BE RELEVANT

| Category* | Citation of document, with indication, where appropriate, of the relevant passages | Relevant to claim No. |
|---|---|---|
| X<br>-<br>A | Buckingham, Julia C. and Cooper, Teresa A., 'Pharmacological characterization of opioid receptors influencing the secretion of corticotropin releasing factor in the rat' Neuroendocrinology (1986), Vol.44, No.1, pp.36-40 | 1,6-9<br><br>2-5 |
| X<br>-<br>A | Fabbri, Andrea, et al. 'Corticotropin-releasing factor is produced by rat Leydig cells and has a major local antireproductive role in the testis' Endocrinology (1990), Vol.127, No.3, pp.1541-3 | 1,6-9<br><br>2-5 |
| X<br>-<br>A | Kamilaris, Themis C., et al., 'Cholecystokinin-octapeptide stimulates hypothalamic-pituitary-adrenal function in rats: role of corticotropin- releasing hormone' Endocrinology (1992), Vol.130, No.4, pp.1764-74 especially, Fig. 8 | 1,6-9<br><br>2-5 |
| X<br>-<br>A | Hashimoto, Kozo, et al., 'Hypothalamic control of glucocorticoid secretion' Shinkei Kenkyu no Shinpo (1998), Vol.42, No.4, pp.678-688, English abstract | 1,6-9<br><br>2-5 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 February, 2001 (13.02.01) | 27 February, 2001 (27.02.01) |
| Name and mailing address of the ISA/<br>Japanese Patent Office | Authorized officer |
| Facsimile No. | Telephone No. |

Form PCT/ISA/210 (second sheet) (July 1992)

INTERNATIONAL SEARCH REPORT

International application No.
PCT/JP00/08119

C (Continuation). DOCUMENTS CONSIDERED TO BE RELEVANT

| Category* | Citation of document, with indication, where appropriate, of the relevant passages | Relevant to claim No. |
|---|---|---|
| X<br>-<br>A | Buckingham, Julia C. and Cooper, Teresa A., 'Differences in hypothalamo-pituitary- adrenocortical activity in the rat after acute and prolonged treatment with morphine'<br>Neuroendocrinology (1984), Vol.38, No.5, pp.411-17 | 1,6-9<br><br>2-5 |
| X<br>-<br>A | Simoncini, T., et al., 'Human umbilical vein endothelial cells: a new source and potential target for corticotropin-releasing factor'<br>J. Clin. Endocrinol. Metab.<br>(August 1999), Vol.84, No.8, pp.2802-2806 | 1,6-9<br><br>2-5 |
| A | Tinajero, J. C., et al., 'Regulation of corticotropin-releasing factor secretion from Leydig cells by serotonin'<br>Endocrinology (1992), Vol.130, No.4, pp.1780-8 | 1-9 |
| A | WO, 97/24436, A2 (TAKEDA CHEMICAL INDUSTRIES, LTD.), 10 July, 1997 (10.07.97),<br>page 155, line 12 to page 157, line 36, example 41<br>& AU, 001208497, A & CA, 2239299, A<br>& EP, 870020, A & JP, 10-146192, A | 1-9 |
| A | EP, 887417, A2 (TAKEDA CHEMICAL INDUSTRIES, LTD.), 30 December, 1998 (30.12.98),<br>page 9, lines 7 to 49<br>& JP, 11-071396, A & CA, 2242086, A1 | 1-9 |

Form PCT/ISA/210 (continuation of second sheet) (July 1992)

## INTERNATIONAL SEARCH REPORT

International application No.
PCT/JP00/08119

**Box I Observations where certain claims were found unsearchable (Continuation of item 1 of first sheet)**

This international search report has not been established in respect of certain claims under Article 17(2)(a) for the following reasons:

1. ☒ Claims Nos.: 10
because they relate to subject matter not required to be searched by this Authority, namely:
Claim 10 pertains to methods for treatment of the human body by therapy and thus relates to a subject matter which this International Searching Authority is not required, under the provisions of Article 17(2)(a)(i) of the PCT and Rule 39.1(iv) of the Regulations under the PCT, to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)